# EUROPEAN PATENT APPLICATION

(11) **EP 4 692 363 A1**
(43) Date of publication of application: **11.02.2026**
(21) Application number: 24780885.0
(22) Date of filing: 29.03.2024
(51) Int. Cl.: C12P 13/12, C12N 1/15, C12N 1/19, C12N 1/21, C12N 5/10, C12N 9/04, C12N 9/14, C12N 9/22, C12N 9/88, C12N 15/31, C12N 15/52, C12N 15/53, C12N 15/54, C12N 15/55, C12N 15/60, C12P 5/00

(54) **METHOD FOR PRODUCING METHYL COMPOUND**

(30) Priority: 31.03.2023 JP 2023056931; 31.03.2023 JP 2023056932
(71) Applicant: Mitsubishi Chemical Corporation, Tokyo 100-8251 (JP)
(72) Inventor: YUNOMURA, Shuichi, Tokyo 100-8251 (JP)
(74) Representative: Hoffmann Eitle
(86) International application number: PCT/JP2024/013239
(87) International publication number: WO 2024/204791

(57) **Abstract**

An object of the present invention is to provide a method for producing a methyl compound. The present invention provides a method for producing a methyl compound using formic acid.

## Description

### [Technical Field]

The present invention relates to a method for producing a methyl compound using formic acid and a method for regenerating S-adenosylmethionine.

### [Background Art]

Various methyltransferases are known in the natural world. These enzymes regioselectively and stereoselectively methylate various compounds as substrates. Most of these methyltransferases transfer a methyl group to target compounds by using S-adenosylmethionine (SAM: S-adenosyl-L-methionine) as a methyl group donor.

SAM serving as a methyl group donor, however, is supplied in a limited amount inside cells and easily depleted.

On the other hand, a method for recycling SAM by adding methanol to a culture medium of a microorganism coexpressing methylase and methanol dehydrogenase is known (Patent Literature 1: International Publication No. WO 2019/160059). This literature states that a microorganism is engineered so as to increase the amount of methionine or SAM, and specifically describes an approach of reducing the activity of formaldehyde dehydrogenase, which directly converts formaldehyde to formic acid, or an approach of reducing the activity of an enzyme for converting formaldehyde to formic acid through binding to glutathione, i.e., an approach of suppressing the conversion of formaldehyde to formic acid, as an approach for reducing formaldehyde-degrading activity.

### [Citation List]

### [Patent Literature]

[Patent Literature 1] International Publication No. WO 2019/160059

### [Summary of Invention]

### [Technical Problem]

A method described in Patent Literature 1 involves constructing a S-adenosylmethionine recycling pathway that supplies methyl group carbon through added methanol, but the method did not have a sufficient accelerating effect on the methylation reaction. There is a demand for providing a novel method that can more efficiently regenerate SAM and accelerate the methylation reaction of a compound. There is also a demand for providing a novel method for producing a methyl compound.

### [Solution to Problem]

The present inventors have succeeded in using formic acid or a salt thereof (hereinafter, also simply referred to as "formic acid") and thereby efficiently regenerating SAM and producing a methyl compound, leading to the completion of the present invention.

Specifically, the present invention is as follows.
[1-1]
   A method for producing a methyl compound, comprising the steps of:
   in the presence of formic acid or a salt thereof,
   (a) producing 5-methyltetrahydrofolate from formic acid or a salt thereof and tetrahydrofolate;
   (b) converting S-adenosylhomocysteine, which is produced by the demethylation of S-adenosylmethionine, to homocysteine;
   (c) producing methionine by transferring a methyl group of the 5-methyltetrahydrofolate to the homocysteine, and converting the methionine to S-adenosylmethionine; and
   (d) performing methylation reaction with S-adenosylmethionine as a methyl group donor to produce a methyl compound.
[1-2]
   The method according to [1-1], wherein in the step (a), the production of 5-methyltetrahydrofolate from formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).
[1-3]
   The method according to [1-1] or [1-2], wherein in the step (b), the conversion of S-adenosylhomocysteine to homocysteine is performed in the presence of S-adenosylhomocysteine nucleosidase (Mtn) and S-ribosylhomocysteine lyase (LuxS).
[1-4]
   The method according to any of [1-1] to [1-3], wherein in the step (b), the conversion of S-adenosylhomocysteine to homocysteine is performed in the presence of S-adenosylhomocysteine hydrolase (SahH).
[1-5]
   The method according to any of [1-1] to [1-4], wherein in the step (b), the demethylation of S-adenosylhomocysteine is performed in the presence of S-adenosylmethionine-dependent methyltransferase.
[1-6]
   The method according to any of [1-1] to [1-5], wherein in the step (c), the production of methionine from the homocysteine is performed in the presence of methionine synthase (MetH or MetE), and the conversion of the methionine to S-adenosylmethionine is performed in the presence of methionine adenosyltransferase (MetK).
[1-7]
   The method according to any of [1-1] to [1-6], further comprising the step of:
   (e) converting methanol to formaldehyde, and converting the formaldehyde to formic acid.
[1-8]
   The method according to [1-7], wherein in the step (e), the conversion of methanol to formaldehyde is performed in the presence of methanol dehydrogenase (Medh) and/or methanol oxidase (Mox).
[1-9]
   The method according to [1-7] or [1-8], wherein in the step (e), the conversion of formaldehyde to formic acid is performed in the presence of formaldehyde dehydrogenase (FdhA).
[1-10]
   The method according to any of [1-7] to [1-9], wherein in the step (e), the conversion of formaldehyde to formic acid is performed in the presence of spontaneous reaction of formaldehyde with glutathione, and in the presence of S-hydroxymethyl glutathione dehydrogenase (FrmA) and S-formylglutathione hydrolase (FrmB).
[1-11]
   The method according to any of [1-1] to [1-10], wherein a cell engineered so as to enhance the activity or expression of S-adenosylmethionine-dependent methyltransferase is used.
[1-12]
   The method according to any of [1-1] to [1-11], wherein the cell is at least one selected from the group consisting of an animal cell, an insect cell, a plant cell, and a microorganism.
[1-13]
   The method according to any of [1-1] to [1-12], wherein the methyl compound is a derivative of an aromatic amino acid.
[1-14]
   A method for producing an organic compound, comprising the step of producing the organic compound via a methyl compound produced by a method according to any of [1-1] to [1-13] as an intermediate.
[1-15]
   A cell engineered so as to enhance the activity or expression of formate-tetrahydrofolate ligase (FtfL) and S-adenosylmethionine-dependent methyltransferase.
[1-16]
   The cell according to [1-15], further engineered so as to enhance the activity or expression of methenyl-tetrahydrofolate cyclohydrolase (Fch) and/or methylene-tetrahydrofolate dehydrogenase (MtdA).
[1-17]
   The cell according to [1-15] or [1-16], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[1-18]
   The cell according to any of [1-15] to [1-17], further engineered so as to enhance the activity or expression of at least one or more enzymes selected from the group consisting of ribose-phosphate diphosphokinase (Prs), adenine phosphoribosyltransferase (Apt), and adenosine kinase (ADK).
[1-19]
   The cell according to any of [1-15] to [1-18], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).
[1-20]
   The cell according to any of [1-15] to [1-19], further engineered so as to enhance the activity or expression of methanol dehydrogenase (Medh) and/or methanol oxidase (Mox).
[1-21]
   The cell according to [1-20], further engineered so as to enhance the activity or expression of at least one or more enzymes selected from the group consisting of formaldehyde dehydrogenase (FdhA), S-hydroxymethyl glutathione dehydrogenase (FrmA), and S-formylglutathione hydrolase (FrmB).
[1-22]
   The cell according to any of [1-15] to [1-21], wherein the cell is at least one selected from the group consisting of an animal cell, an insect cell, a plant cell, and a microorganism.
[1-23]
   A method for regenerating S-adenosylmethionine, comprising the steps of:
   (a) producing 5-methyltetrahydrofolate from formic acid or a salt thereof and tetrahydrofolate;
   (b) converting S-adenosylhomocysteine produced by the demethylation of S-adenosylmethionine to homocysteine; and
   (c) producing methionine by transferring a methyl group of the 5-methyltetrahydrofolate to the homocysteine, and converting the methionine to S-adenosylmethionine.
[2-1]
   A method for regenerating S-adenosylmethionine, comprising the steps of:
   (a) producing 5-methyltetrahydrofolate from formic acid or a salt thereof and tetrahydrofolate;
   (b) converting S-adenosylhomocysteine, which is produced by the demethylation of S-adenosylmethionine, to homocysteine; and
   (c) producing methionine by transferring a methyl group of the 5-methyltetrahydrofolate to the homocysteine, and converting the methionine to S-adenosylmethionine.
[2-2]
   The method for regenerating S-adenosylmethionine according to [2-1], wherein in the step (a), the production of 5-methyltetrahydrofolate from formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).
[2-3]
   The method for regenerating S-adenosylmethionine according to [2-1] or [2-2], wherein in the step (b), the conversion of S-adenosylhomocysteine to homocysteine is performed in the presence of S-adenosylhomocysteine nucleosidase (Mtn) and S-ribosylhomocysteine lyase (LuxS).
[2-4]
   The method for regenerating S-adenosylmethionine according to any of [2-1] to [2-3], wherein in the step (b), the conversion of S-adenosylhomocysteine to homocysteine is performed in the presence of S-adenosylhomocysteine hydrolase (SahH).
[2-5]
   The method for regenerating S-adenosylmethionine according to any of [2-1] to [2-4], wherein in the step (b), the demethylation of S-adenosylhomocysteine is performed in the presence of S-adenosylmethionine-dependent methyltransferase.
[2-6]
   The method for regenerating S-adenosylmethionine according to any of [2-1] to [2-5], wherein in the step (c), the production of methionine from the homocysteine is performed in the presence of methionine synthase (MetH or MetE), and the conversion of the methionine to S-adenosylmethionine is performed in the presence of methionine adenosyltransferase (MetK).
[2-7]
   The method for regenerating S-adenosylmethionine according to any of [2-1] to [2-6], wherein a cell engineered so as to enhance the activity or expression of S-adenosylmethionine-dependent methyltransferase is used.
[2-8]
   The method for regenerating S-adenosylmethionine according to any of [2-1] to [2-7], wherein the cell is at least one selected from the group consisting of an animal cell, an insect cell, a plant cell, and a microorganism.
[2-9]
   A method for producing a methyl compound, comprising the steps of:
   (a) producing 5-methyltetrahydrofolate from formic acid or a salt thereof and tetrahydrofolate;
   (b) converting S-adenosylhomocysteine, which is produced by the demethylation of S-adenosylmethionine, to homocysteine;
   (c) producing methionine by transferring a methyl group of the 5-methyltetrahydrofolate to the homocysteine, and converting the methionine to S-adenosylmethionine; and
   (d) performing methylation reaction with S-adenosylmethionine as a methyl group donor to produce a methyl compound.
[2-10]
   The method for producing a methyl compound according to [2-9], wherein in the step (a), the production of 5-methyltetrahydrofolate from formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).
[2-11]
   The method for producing a methyl compound according to [2-9] or [2-10], wherein in the step (b), the conversion of S-adenosylhomocysteine to homocysteine is performed in the presence of S-adenosylhomocysteine nucleosidase (Mtn) and S-ribosylhomocysteine lyase (LuxS).
[2-12]
   The method for producing a methyl compound according to any of [2-9] to [2-11], wherein in the step (b), the conversion of S-adenosylhomocysteine to homocysteine is performed in the presence of S-adenosylhomocysteine hydrolase (SahH).
[2-13]
   The method for producing a methyl compound according to any of [2-9] to [2-12], wherein in the step (b), the demethylation of S-adenosylhomocysteine is performed in the presence of S-adenosylmethionine-dependent methyltransferase.
[2-14]
   The method for producing a methyl compound according to any of [2-9] to [2-13], wherein in the step (c), the production of methionine from the homocysteine is performed in the presence of methionine synthase (MetH or MetE), and the conversion of the methionine to S-adenosylmethionine is performed in the presence of methionine adenosyltransferase (MetK).
[2-15]
   The method for producing a methyl compound according to any of [2-9] to [2-14], wherein a cell engineered so as to enhance the activity or expression of S-adenosylmethionine-dependent methyltransferase is used.
[2-16]
   The method for producing a methyl compound according to any of [2-9] to [2-15], wherein the cell is at least one selected from the group consisting of an animal cell, an insect cell, a plant cell, and a microorganism.
[2-17]
   A method for producing an organic compound, comprising the step of producing the organic compound via a methyl compound produced by a method according to any of [2-9] to [2-16] as an intermediate.
[2-18]
   A cell engineered so as to enhance the activity or expression of formate-tetrahydrofolate ligase (FtfL) and S-adenosylmethionine-dependent methyltransferase.
[2-19]
   The cell according to [2-18], further engineered so as to enhance the activity or expression of methenyl-tetrahydrofolate cyclohydrolase (Fch) and/or methylene-tetrahydrofolate dehydrogenase (MtdA).
[2-20]
   The cell according to [2-18] or [2-19], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[2-21]
   The cell according to any of [2-18] to [2-20], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).
[2-22]
   The cell according to any of [2-18] to [2-21], wherein the cell is at least one selected from the group consisting of an animal cell, an insect cell, a plant cell, and a microorganism.
[2-23]
   A method for producing a methyl compound, comprising using a cell according to any of [2-18] to [2-22].
[2-24]
   A method for producing an organic compound, comprising the step of producing the organic compound via a methyl compound produced using a cell according to any of [2-18] to [2-22] as an intermediate.
[3-1]
   A method for regenerating S-adenosylmethionine, comprising the steps of:
   (a) converting methanol to formaldehyde, and converting the formaldehyde to formic acid;
   (b) producing 5-methyltetrahydrofolate from the formic acid and tetrahydrofolate;
   (c) converting S-adenosylhomocysteine, which is produced by the demethylation of S-adenosylmethionine, to homocysteine; and
   (d) producing methionine by transferring a methyl group of the 5-methyltetrahydrofolate to the homocysteine, and converting the methionine to S-adenosylmethionine.
[3-2]
   The method for regenerating S-adenosylmethionine according to [3-1], wherein in the step (a), the conversion of methanol to formaldehyde is performed in the presence of methanol dehydrogenase (Medh) and/or methanol oxidase (Mox).
[3-3]
   The method for regenerating S-adenosylmethionine according to [3-1] or [3-2], wherein in the step (a), the conversion of the formaldehyde to formic acid is performed in the presence of formaldehyde dehydrogenase (FdhA).
[3-4]
   The method for regenerating S-adenosylmethionine according to any of [3-1] to [3-3], wherein in the step (a), the conversion of the formaldehyde to formic acid is performed in the presence of spontaneous reaction of formaldehyde with glutathione, and in the presence of S-hydroxymethyl glutathione dehydrogenase (FrmA) and S-formylglutathione hydrolase (FrmB).
[3-5]
   The method for regenerating S-adenosylmethionine according to any of [3-1] to [3-4], wherein in the step (b), the production of 5-methyltetrahydrofolate from formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).
[3-6]
   The method for regenerating S-adenosylmethionine according to any of [3-1] to [3-5], wherein in the step (c), the conversion of S-adenosylhomocysteine to homocysteine is performed in the presence of S-adenosylhomocysteine nucleosidase (Mtn) and S-ribosylhomocysteine lyase (LuxS).
[3-7]
   The method for regenerating S-adenosylmethionine according to any of [3-1] to [3-6], wherein in the step (c), the conversion of S-adenosylhomocysteine to homocysteine is performed in the presence of S-adenosylhomocysteine hydrolase (SahH).
[3-8]
   The method for regenerating S-adenosylmethionine according to any of [3-1] to [3-7], wherein in the step (c), the demethylation of S-adenosylhomocysteine is performed in the presence of S-adenosylmethionine-dependent methyltransferase.
[3-9]
   The method for regenerating S-adenosylmethionine according to any of [3-1] to [3-8], wherein in the step (d), the production of methionine from the homocysteine is performed in the presence of methionine synthase (MetH or MetE), and the conversion of the methionine to S-adenosylmethionine is performed in the presence of methionine adenosyltransferase (MetK).
[3-10]
   The method for regenerating S-adenosylmethionine according to any of [3-1] to [3-9], wherein a cell engineered so as to enhance the activity or expression of S-adenosylmethionine-dependent methyltransferase is used.
[3-11]
   The method for regenerating S-adenosylmethionine according to any of [3-1] to [3-10], wherein the cell is at least one selected from the group consisting of an animal cell, an insect cell, a plant cell, and a microorganism.
[3-12]
   A method for producing a methyl compound, comprising the steps of:
   (a) converting methanol to formaldehyde, and converting the formaldehyde to formic acid;
   (b) producing 5-methyltetrahydrofolate from the formic acid and tetrahydrofolate;
   (c) converting S-adenosylhomocysteine, which is produced by the demethylation of S-adenosylmethionine, to homocysteine;
   (d) producing methionine by transferring a methyl group of the 5-methyltetrahydrofolate to the homocysteine, and converting the methionine to S-adenosylmethionine; and
   (e) performing methylation reaction with S-adenosylmethionine as a methyl group donor to produce a methyl compound.
[3-13]
   The method for producing a methyl compound according to [3-12], wherein in the step (a), the conversion of methanol to formaldehyde is performed in the presence of methanol dehydrogenase (Medh) and/or methanol oxidase (Mox).
[3-14]
   The method for producing a methyl compound according to [3-12] or [3-13], wherein in the step (a), the conversion of the formaldehyde to formic acid is performed in the presence of formaldehyde dehydrogenase (FdhA).
[3-15]
   The method for producing a methyl compound according to any of [3-12] to [3-14], wherein in the step (a), the conversion of the formaldehyde to formic acid is performed in the presence of spontaneous reaction of formaldehyde with glutathione, and in the presence of S-hydroxymethyl glutathione dehydrogenase (FrmA) and S-formylglutathione hydrolase (FrmB).
[3-16]
   The method for producing a methyl compound according to any of [3-12] to [3-15], wherein in the step (b), the production of 5-methyltetrahydrofolate from the formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).
[3-17]
   The method for producing a methyl compound according to any of [3-12] to [3-16], wherein in the step (c), the conversion of S-adenosylhomocysteine to homocysteine is performed in the presence of S-adenosylhomocysteine nucleosidase (Mtn) and S-ribosylhomocysteine lyase (LuxS).
[3-18]
   The method for producing a methyl compound according to any of [3-12] to [3-17], wherein in the step (c), the conversion of S-adenosylhomocysteine to homocysteine is performed in the presence of S-adenosylhomocysteine hydrolase (SahH).
[3-19]
   The method for producing a methyl compound according to any of [3-12] to [3-18], wherein in the step (c), the demethylation of S-adenosylhomocysteine is performed in the presence of S-adenosylmethionine-dependent methyltransferase.
[3-20]
   The method for producing a methyl compound according to any of [3-12] to [3-19], wherein in the step (d), the production of methionine from the homocysteine is performed in the presence of methionine synthase (MetH or MetE), and the conversion of the methionine to S-adenosylmethionine is performed in the presence of methionine adenosyltransferase (MetK).
[3-21]
   The method for producing a methyl compound according to any of [3-12] to [3-20], wherein a cell engineered so as to enhance the activity or expression of S-adenosylmethionine-dependent methyltransferase is used.
[3-22]
   The method for producing a methyl compound according to any of [3-12] to [3-21], wherein the cell is at least one selected from the group consisting of an animal cell, an insect cell, a plant cell, and a microorganism.
[3-23]
   A method for producing an organic compound, comprising the step of producing the organic compound via a methyl compound produced by a method according to any of [3-12] to [3-22] as an intermediate.
[3-24]
   A cell engineered so as to enhance the activity or expression of methanol dehydrogenase (Medh) and/or methanol oxidase (Mox), formate-tetrahydrofolate ligase (FtfL), and S-adenosylmethionine-dependent methyltransferase.
[3-25]
   The cell according to [3-24], further engineered so as to enhance the activity or expression of at least one or more enzymes selected from the group consisting of formaldehyde dehydrogenase (FdhA), S-hydroxymethyl glutathione dehydrogenase (FrmA), and S-formylglutathione hydrolase (FrmB).
[3-26]
   The cell according to [3-24] or [3-25], further engineered so as to enhance the activity or expression of methenyl-tetrahydrofolate cyclohydrolase (Fch) and/or methylene-tetrahydrofolate dehydrogenase (MtdA).
[3-27]
   The cell according to any of [3-24] to [3-26], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[3-28]
   The cell according to any of [3-24] to [3-27], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).
[3-29]
   The cell according to any of [3-24] to [3-28], wherein the cell is at least one selected from the group consisting of an animal cell, an insect cell, a plant cell, and a microorganism.
[3-30]
   A method for producing a methyl compound, comprising using a cell according to any of [3-24] to [3-29].
[3-31]
   A method for producing an organic compound, comprising the step of producing the organic compound via a methyl compound produced using a cell according to any of [3-24] to [3-29] as an intermediate.
[3-32]
   A method for regenerating S-adenosylmethionine, comprising the steps of:
   (a) converting methanol to formaldehyde;
   (b) producing 5-methyltetrahydrofolate from the formaldehyde and tetrahydrofolate;
   (c) converting S-adenosylhomocysteine, which is produced by the demethylation of S-adenosylmethionine, to homocysteine; and
   (d) producing methionine by transferring a methyl group of the 5-methyltetrahydrofolate to the homocysteine, and converting the methionine to S-adenosylmethionine.
[3-33]
   The method for regenerating S-adenosylmethionine according to [3-32], wherein in the step (a), the conversion of methanol to formaldehyde is performed in the presence of methanol dehydrogenase (Medh) and/or methanol oxidase (Mox).
[3-34]
   The method for regenerating S-adenosylmethionine according to [3-32] or [3-33], wherein in the step (b), the production of 5-methyltetrahydrofolate from the formaldehyde and tetrahydrofolate is performed in the presence of 5,10-methylene-tetrahydrofolate reductase (MetF).
[3-35]
   The method for regenerating S-adenosylmethionine according to any of [3-32] to [3-34], wherein in the step (c), the conversion of S-adenosylhomocysteine to homocysteine is performed in the presence of S-adenosylhomocysteine nucleosidase (Mtn) and S-ribosylhomocysteine lyase (LuxS).
[3-36]
   The method for regenerating S-adenosylmethionine according to any of [3-32] to [3-35], wherein in the step (c), the conversion of S-adenosylhomocysteine to homocysteine is performed in the presence of S-adenosylhomocysteine hydrolase (SahH).
[3-37]
   The method for regenerating S-adenosylmethionine according to any of [3-32] to [3-36], wherein in the step (c), the demethylation of S-adenosylhomocysteine is performed in the presence of S-adenosylmethionine-dependent methyltransferase.
[3-38]
   The method for regenerating S-adenosylmethionine according to any of [3-32] to [3-37], wherein in the step (d), the production of methionine from the homocysteine is performed in the presence of methionine synthase (MetH or MetE), and the conversion of the methionine to S-adenosylmethionine is performed in the presence of methionine adenosyltransferase (MetK).
[3-39]
   The method for regenerating S-adenosylmethionine according to any of [3-32] to [3-38], wherein a cell engineered so as to enhance the activity or expression of S-adenosylmethionine-dependent methyltransferase is used.
[3-40]
   The method for regenerating S-adenosylmethionine according to any of [3-32] to [3-39], wherein the cell is at least one selected from the group consisting of an animal cell, an insect cell, a plant cell, and a microorganism.
[3-41]
   A method for producing a methyl compound, comprising the steps of:
   (a) converting methanol to formaldehyde;
   (b) producing 5-methyltetrahydrofolate from the formaldehyde and tetrahydrofolate;
   (c) converting S-adenosylhomocysteine, which is produced by the demethylation of S-adenosylmethionine, to homocysteine;
   (d) producing methionine by transferring a methyl group of the 5-methyltetrahydrofolate to the homocysteine, and converting the methionine to S-adenosylmethionine; and
   (e) performing methylation reaction with the S-adenosylmethionine produced in the step (d) as a methyl group donor to produce a methyl compound.
[3-42]
   The method for producing a methyl compound according to [3-41], wherein in the step (a), the conversion of methanol to formaldehyde is performed in the presence of methanol dehydrogenase (Medh) and/or methanol oxidase (Mox).
[3-43]
   The method for producing a methyl compound according to [3-41] or [3-42], wherein in the step (b), the production of 5-methyltetrahydrofolate from the formaldehyde and tetrahydrofolate is performed in the presence of 5,10-methylene-tetrahydrofolate reductase (MetF).
[3-44]
   The method for producing a methyl compound according to any of [3-41] to [3-43], wherein in the step (c), the conversion of S-adenosylhomocysteine to homocysteine is performed in the presence of S-adenosylhomocysteine nucleosidase (Mtn) and S-ribosylhomocysteine lyase (LuxS).
[3-45]
   The method for producing a methyl compound according to any of [3-41] to [3-44], wherein in the step (c), the conversion of S-adenosylhomocysteine to homocysteine is performed in the presence of S-adenosylhomocysteine hydrolase (SahH).
[3-46]
   The method for producing a methyl compound according to any of [3-41] to [3-45], wherein in the step (c), the demethylation of S-adenosylhomocysteine is performed in the presence of S-adenosylmethionine-dependent methyltransferase.
[3-47]
   The method for producing a methyl compound according to any of [3-41] to [3-46], wherein in the step (d), the production of methionine from the homocysteine is performed in the presence of methionine synthase (MetH or MetE), and the conversion of the methionine to S-adenosylmethionine is performed in the presence of methionine adenosyltransferase (MetK).
[3-48]
   The method for producing a methyl compound according to any of [3-41] to [3-47], wherein a cell engineered so as to enhance the activity or expression of S-adenosylmethionine-dependent methyltransferase is used.
[3-49]
   The method for producing a methyl compound according to any of [3-41] to [3-48], wherein the cell is at least one selected from the group consisting of an animal cell, an insect cell, a plant cell, and a microorganism.
[3-50]
   A method for producing an organic compound, comprising the step of producing the organic compound via a methyl compound produced by a method according to any of [3-41] to [3-49] as an intermediate.
[3-51]
   A cell engineered so as to enhance the activity or expression of methanol dehydrogenase (Medh) and/or methanol oxidase (Mox) and S-adenosylmethionine-dependent methyltransferase.
[3-52]
   The cell according to [3-51], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[3-53]
   The cell according to [3-51] or [3-52], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).
[3-54]
   The cell according to any of [3-51] to [3-53], wherein the cell is at least one selected from the group consisting of an animal cell, an insect cell, a plant cell, and a microorganism.
[3-55]
   A method for producing a methyl compound, comprising using a cell according to any of [3-51] to [3-54].
[3-56]
   A method for producing an organic compound, comprising the step of producing the organic compound via a methyl compound produced using a cell according to any of [3-51] to [3-54] as an intermediate.

### [Advantageous Effects of Invention]

According to the present invention, SAM can be efficiently regenerated.

### [Brief Description of Drawings]

[Figure 1] Figure 1 is a diagram showing one example of a reaction system for use in the method of the present invention.
[Figure 2] Figure 2 is a diagram showing one example of a reaction system for use in the method of the present invention.

### [Description of Embodiments]

Hereinafter, the present invention will be described in detail. Embodiments given below are illustrations for describing the present invention, and the present invention is not intended to be limited to only these embodiments. The present invention can be carried out in various forms without departing from the spirit of the present invention.

The present specification encompasses the contents described in the specifications and drawings of Japanese Patent Application Nos. 2023-056931 and 2023-056932 filed on March 31, 2023, based on which the priority of the present application is claimed.

### 1. Summary

SAM is useful in the methylation reaction of a compound, but inside cells, it is supplied in a limited amount and easily depleted.

A method for recycling SAM by adding methanol to a culture medium of a microorganism coexpressing methylase and methanol dehydrogenase has heretofore been known. For this method, spontaneous condensation reaction of formaldehyde with tetrahydrofolate (THF) is essential, and the literature describing this method (International Publication No. WO 2019/160059) lists an approach of reducing the activity of formaldehyde dehydrogenase, which directly converts formaldehyde to formic acid, or an approach of reducing the activity of an enzyme for converting formaldehyde to formic acid through binding to glutathione, i.e., an approach of suppressing the conversion of formaldehyde to formic acid, as an approach for reducing formaldehyde-degrading activity.

Against this background, the present inventor has succeeded in rather using formic acid in SAM regeneration or rather using a reaction pathway that converts formaldehyde to formic acid and thereby efficiently regenerating SAM, leading to the completion of the present invention.

### 2-1. Method for regenerating S-adenosylmethionine using formic acid

### (1) Formic acid

Formic acid is a carboxylic acid having the smallest molecular weight and is a compound represented by the chemical formula HCOOH. In the present invention, the formic acid can be used in the form of formic acid or a salt thereof. Examples of the salt of the formic acid (formate) include, but are not limited to, potassium formate, sodium formate, lithium formate, cesium formate, thallous formate, and ammonium formate. In the present invention, a hydrate of the formic acid or the salt thereof may be used as the formic acid.

### (2) S-Adenosylmethionine

S-Adenosylmethionine (SAM; S-adenosyl-L-methionine) is a substance that functions as a methyl group donor when methyltransferase transfers a methyl group to a methylation target substance (substance to be methylated). SAM becomes S-adenosyl-L-homocysteine (SAH) when losing its methyl group (demethylated) by transferring the methyl group to a methylation target substance. SAH is degraded into homocysteine (Hcy) through S-ribosylhomocysteine (SRH). Since SAM is supplied in a limited amount inside cells, SAM is easily depleted unless methionine or SAM is newly supplied.

### (3) Method for regenerating S-Adenosylmethionine

As used herein, the "regeneration" of SAM means that SAM is produced without adding methionine or SAM from the outside of the system. Those skilled in the art can evaluate whether or not SAM has been produced by use of a known method, for example, a method of measuring methyltransferase activity in a test sample. For example, SAM can be evaluated by measuring methyltransferase activity in a test sample and determining that it has been produced when the activity in the test sample is higher than that of a control. A sample differing in one condition from the test sample, for example, a sample that is not supplemented with a substance to be added to the test sample, can be used as the control. The methyltransferase activity can be evaluated, for example, by introducing foreign methyltransferase gene to a cell, inducing the expression of the enzyme, then adding a methylation target substance to a cell culture solution so as to react with the enzyme, and measuring the amount of a methyl compound produced by mass spectrometry such as LC-MS. Specifically, when the methyltransferase activity in the test sample is higher than that of a control without adding methionine or SAM from the outside of the reaction system, it can be concluded that SAM has been regenerated in this test sample. However, in the present invention, the method for measuring methyltransferase activity is not limited to the method described above, and those skilled in the art can appropriately adopt a known method.

The phrase "higher than that of a control" means that the methyltransferase activity is higher by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% or more than that of a control.

The method for regenerating S-adenosylmethionine according to the present invention can comprise, for example, the steps of:
(a) producing 5-methyltetrahydrofolate from formic acid or a salt thereof and tetrahydrofolate;
(b) converting S-adenosylhomocysteine, which is produced by the demethylation of S-adenosylmethionine, to homocysteine; and
(c) producing methionine by transferring a methyl group of the 5-methyltetrahydrofolate to the homocysteine, and converting the methionine to S-adenosylmethionine.

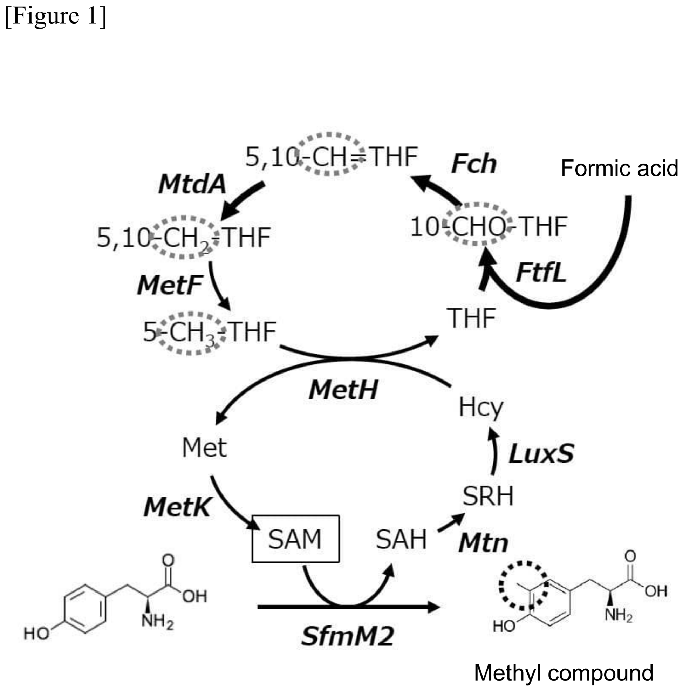

The step (a) is the step of producing 5-methyltetrahydrofolate (5-CH₃-THF) from formic acid or a salt thereof and tetrahydrofolate (THF) (Figure 1).

The step (a) can comprise, for example, the steps of: (i) producing 10-formyltetrahydrofolate (10-CHO-THF) from formic acid or a salt thereof and tetrahydrofolate (THF); (ii) producing 5,10-methenyl-tetrahydrofolate (5,10-CH=THF) from 10-CHO-THF; (iii) producing 5,10-methylene-tetrahydrofolate (5,10-CH₂-THF) from 5,10-CH=THF; and (iv) producing 5-methyltetrahydrofolate (5-CH₃-THF) from 5,10-CH₂-THF.

In the step (i), the production of 10-CHO-THF from formic acid or a salt thereof and tetrahydrofolate (THF) can be performed in the presence of, for example, formate-tetrahydrofolate ligase (FtfL) or with this enzyme.

In the step (ii), the production of 5,10-CH=THF from 10-CHO-THF can be performed in the presence of, for example, methenyl-tetrahydrofolate cyclohydrolase (Fch) or with this enzyme.

In the step (iii), the production of 5,10-CH₂-THF from 5,10-CH=THF can be performed in the presence of, for example, methylene-tetrahydrofolate dehydrogenase (MtdA) or with this enzyme.

In the step (iv), the production of 5-CH₃-THF from 5,10-CH₂-THF can be performed in the presence of, for example, 5,10-methylene-tetrahydrofolate reductase (MetF) or with this enzyme.

Specifically, in the step (a), the production of 5-methyltetrahydrofolate from formic acid and tetrahydrofolate can be performed in the presence of, for example, formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF) or with these enzymes.

The step (b) is the step of converting S-adenosylhomocysteine (SAH) produced by the demethylation of S-adenosylmethionine (SAM) to homocysteine (Hcy) (Figure 1).

The step (b) can comprise, for example, the steps of: (i) converting S-adenosylhomocysteine (SAH) to S-ribosylhomocysteine (SRH); and (ii) converting SRH to homocysteine (Hcy).

In the step (i), the conversion of S-adenosylhomocysteine (SAH) to S-ribosylhomocysteine (SRH) can be performed in the presence of, for example, S-adenosylhomocysteine nucleosidase (Mtn) or with this enzyme.

In the step (ii), the conversion of SRH to homocysteine (Hcy) can be performed in the presence of, for example, S-ribosylhomocysteine lyase (LuxS) or with this enzyme.

Specifically, in the step (b), the conversion of S-adenosylhomocysteine to homocysteine can be performed in the presence of S-adenosylhomocysteine nucleosidase (Mtn) and S-ribosylhomocysteine lyase (LuxS) or with these enzymes.

In another aspect, the step (b) can comprise, for example, the step of (iii) directly (without conversion to (S-ribosylhomocysteine (SRH)) converting S-adenosylhomocysteine (SAH) to homocysteine (Hcy).

In the step (iii), the conversion of S-adenosylhomocysteine to homocysteine can be performed in the presence of S-adenosylhomocysteine hydrolase (SahH) or with this enzyme.

In the step (b), the demethylation of S-adenosylmethionine (SAM) can be performed in the presence of, for example, S-adenosylmethionine-dependent methyltransferase or with this enzyme.

S-Adenosylmethionine-dependent methyltransferase is an enzyme that transfers a methyl group of SAM to a methylation target substance (substance to be methylated). Examples of the methylation target substance include DNAs, proteins, and low-molecular compounds that are subject to methylation. A low-molecular compound is preferred. SAM is demethylated by S-adenosylmethionine-dependent methyltransferase and thereby converted to SAH. On the other hand, the methylation target substance is methylated to thereby produce a methyl compound. In the present specification, the S-adenosylmethionine-dependent methyltransferase is also simply referred to as "methyltransferase."

In the present invention, the methyltransferase is not limited as long as the methyltransferase transfers a methyl group of SAM to a methylation target substance. Examples of such methyltransferase include, but are not limited to, methyltransferase that methylates a DNA, methyltransferase that methylates a protein, and a transferase that methylates a low-molecular compound. Alternatively, the methyltransferase can be obtained, for example, by searching a known database BRENDA (https://www.brenda-enzyme) for "methyltransferase" as a keyword and selecting an arbitrary enzyme described as "S-adenosyl-L-methionine" in the section "Synonyms." Examples of such methyltransferase include, but are not limited to, L-tyrosine C3-methyltransferase, L-histidine N-alpha-methyltransferase, perillyl acid O-methyltransferase, and catechol O-methyltransferase.

The step (c) is the step of producing methionine by transferring a methyl group of the 5-methyltetrahydrofolate produced in the step (a) to the homocysteine (Hcy) obtained by the conversion of S-adenosylhomocysteine (SAH) in the step (b), and converting the produced methionine to S-adenosylmethionine (SAM). SAM is regenerated by this step.

The step (c) can comprise, for example, the steps of: (i) producing methionine by transferring a methyl group of the 5-methyltetrahydrofolate produced in the step (a) to the homocysteine (Hcy) obtained by the conversion of S-adenosylhomocysteine (SAH) in the step (b); and (ii) converting the produced methionine to S-adenosylmethionine (SAM).

In the step (i), the production of methionine from the homocysteine can be performed in the presence of, for example, methionine synthase (MetH or MetE) or with this enzyme.

In the step (ii), the conversion of the methionine to S-adenosylmethionine can be performed in the presence of, for example, methionine adenosyltransferase (MetK) or with this enzyme.

Specifically, the production of methionine from the homocysteine can be performed in the presence of methionine synthase (MetH or MetE) or with this enzyme, and the conversion of the methionine to S-adenosylmethionine can be performed in the presence of methionine adenosyltransferase (MetK) or with this enzyme. The methionine synthase may be any of a cobalamin-dependent form (MetH) and a cobalamin-independent form (MetE).

In the step (ii), the conversion of the methionine to S-adenosylmethionine requires adenosine triphosphate (ATP). This ATP can be synthesized by a step comprising the reaction of at least one or more enzymes selected from the group consisting of ribose-phosphate diphosphokinase (Prs), adenine phosphoribosyltransferase (Apt), and adenosine kinase (ADK).

Ribose-phosphate diphosphokinase (Prs) can synthesize phosphoribosyl pyrophosphate (PRPP) from ribose-5-phosphate (R5P), a metabolite of the pentose phosphate pathway. Adenine phosphoribosyltransferase (Apt) can synthesize adenosine monophosphate (AMP) from PRPP and adenine. Adenosine kinase (ADK) can synthesize adenosine monophosphate (AMP) from adenosine. AMP can be further phosphorylated and thereby converted to ATP.

Adenine is a metabolite resulting from the reaction of Mtn in the step (b), and adenosine is a metabolite resulting from the reaction of SahH in the step (b). These metabolites are utilized as substrates for the synthesis of ATP so that ATP necessary for the synthesis of SAM can be efficiently supplied.

These enzymes for use in the method of the present invention, in the case of using a cell in the regeneration of SAM, may be, for example, endogenous enzymes of the cell or may be expressed by introducing foreign DNAs encoding the enzymes to the cell.

Those skilled in the art can appropriately set the reaction conditions (a temperature, a reaction time, pH, etc.) of each enzyme in the method of the present invention depending on the type of the enzyme, the type of the cell used, and the like. The temperature condition is, for example, but not limited to, approximately 25 to 40°C, for example, approximately 30 to 37°C. The reaction time is, for example, but not limited to, approximately 15 minutes to 36 hours, for example, approximately 30 minutes to 24 hours (e.g., 1, 2, 4, 6, 8, 10, 12, 16, 18, or 24 hours). The pH can be appropriately set within the range of approximately 6 to 9.

Those skilled in the art can perform each step described above in a cell-free system by a known approach (Microbial Cell Factories, Volume 11, Article number 120, September 2012, Journal of Bioscience and Bioengineering, Volume 129, Issue 3, March 2020, Pages 269-275).

### (4) Cell

The method for regenerating S-adenosylmethionine according to the present invention can be performed using a cell (e.g., a microorganism) (in a cell (e.g., a microorganism)). The method of the present invention can employ any cell containing SAM. Therefore, the cell used in the present invention is not limited as long as SAM exists in the cell. Examples thereof include microorganisms, mammalian cells, insect cells, and plant cells (including cultured plant cells). Examples of the microorganism include, but are not limited to, *E. coli,* coryneform bacteria, bacteria of the genus *Bacillus,* bacteria of the genus *Pantoea,* bacteria of the genus *Enterobacter,* bacteria of the genus *Pseudomonas,* bacteria of the genus *Streptomyces,* bacteria of the genus *Actinomyces,* filamentous fungi, and yeasts. Examples of the mammalian cell include, but are not limited to, mouse cells, rat cells, rabbit cells, dog cells, monkey cells, and human-derived cultured cells. The insect cell is not limited as long as the cell permits protein expression by baculovirus or the like. Examples of the plant cell include, but are not limited to, cells of plants of the genus *Nicotiana* (e.g., *Nicotiana benthamiana, N. tabacum,* and *N. excelsior),* plants of the family *Solanaceae,* plants of the family *Poaceae,* plants of the family *Brassicaceae,* plants of the family *Compositae,* and bryophytes. The cell used in the present invention is preferably a cell capable of expressing a foreign gene.

A cell engineered so as to enhance the activity or expression of S-adenosylmethionine-dependent methyltransferase can be used in the present invention. As used herein, the "cell engineered" includes a "microorganism engineered."

In the present invention, a cell engineered so as to enhance the activity or expression of formate-tetrahydrofolate ligase (FtfL) and S-adenosylmethionine-dependent methyltransferase can be used.

In the present invention, a cell further engineered so as to enhance the activity or expression of methenyl-tetrahydrofolate cyclohydrolase (Fch) and/or methylene-tetrahydrofolate dehydrogenase (MtdA) in addition to FtfL and SAM-dependent methyltransferase can be further used.

In the present invention, a cell further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH) (e.g., Mtn and LuxS) in addition to FtfL and SAM-dependent methyltransferase can be further used.

In the present invention, a cell engineered so as to enhance the activity or expression of at least one or more enzymes selected from the group consisting of ribose-phosphate diphosphokinase (Prs), adenine phosphoribosyltransferase (Apt), and adenosine kinase (ADK) (e.g., Prs and Apt) in addition to FtfL and SAM-dependent methyltransferase can be further used.

In the present invention, a cell engineered so as to enhance the activity or expression of at least one or more enzymes selected from the group consisting of the following enzymes (i) to (iv):
(i) Fch and/or MtdA,
(ii) at least one or more enzymes selected from the group consisting of Mtn, LuxS, and SahH,
(iii) at least one or more enzymes selected from the group consisting of Prs, Apt, and ADK (e.g., Prs and Apt or only ADK), and
(iv) at least one or more enzymes selected from the group consisting of 5,10-methylene-tetrahydrofolate reductase (MetF), methionine synthase (MetH or MetE), and methionine adenosyltransferase (MetK)
in addition to FtfL and SAM-dependent methyltransferase can be further used.

As used herein, the phrase "engineered so as to enhance the activity or expression" of an enzyme means being engineered so as to enhance the activity or expression of the enzyme by, for example, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% or more as compared with the activity or expression of the enzyme in a non-engineered cell (cell that has not been engineered). Whether or not the activity or expression of the enzyme is enhanced can be evaluated by measuring the expression level of the enzyme in the engineered cell and examining the presence or absence of increase in the expression level as compared with a non-engineered cell.

Examples of the cell engineered so as to enhance the activity or expression of SAM-dependent methyltransferase include cells comprising a DNA encoding SAM-dependent methyltransferase. The SAM-dependent methyltransferase can be appropriately selected on the basis of a known database, for example, BRENDA (https://www.brenda-enzyme), as described above.

Examples of the cell engineered so as to enhance the activity or expression of each of FtfL, Fch, MtdA, Mtn, LuxS, SahH, Prs, Apt, ADK, MetF, MetH, MetE, and MetK include cells comprising a DNA encoding each of FtfL, Fch, MtdA, Mtn, LuxS, SahH, Prs, Apt, ADK, MetF, MetH, MetE, and MetK.

In the present invention, the "cell engineered" and the cell comprising a DNA encoding each enzyme can also be referred to as a "transformant."

The DNA encoding each of SAM-dependent methyltransferase, FtfL, Fch, MtdA, Mtn, LuxS, SahH, Prs, Apt, ADK, MetF, MetH, MetE, and MetK can be prepared by obtaining information on the nucleotide sequence thereof from a known database, for example, NCBI (https://www.ncbi.nlm.nih.gov/nucleotide/), and using a known genetic engineering approach.

In the present invention, a vector comprising the DNA encoding each enzyme can be used for transfecting a cell with the DNA. Examples of such a vector include plasmid vectors and virus vectors. Those skilled in the art can appropriately select the type of the vector depending on the type of a host cell and a purpose. The vector of the present invention can comprise, in addition to the DNA encoding the enzyme, cis elements such as a promoter and an enhancer, a splicing signal, a poly-A addition signal, a ribosomal binding sequence (SD sequence), a selective marker gene, a reporter gene, and the like, and these elements may be engineered.

The vector of the present invention is not limited as long as the enzyme used in the present invention can be expressed by a host cell. For example, a vector that enables two or more enzyme genes to be expressed can be used. Examples of such a vector, in the case of a plasmid vector, include, but are not limited to, pET Duet-1, pCOLA Duet-1, pACYC Duet-1, and pCDF Duet-1 (Novagen).

In the present invention, the vector can comprise DNAs encoding one or more enzymes. For example, one vector may comprise DNAs encoding FtfL, Fch, and MtdA, DNAs encoding MetF, MetH, and MetK, DNAs encoding Mtn and LuxS, or DNAs encoding MetF, MetH, MetK, Mtn, and LuxS.

In the present invention, for example, a formic acid fixation pathway that produces 5-methyltetrahydrofolate from formic acid can be introduced to a cell by introducing DNAs encoding FtfL, Fch, and MtdA to the cell. Specifically, a cell having a formic acid fixation pathway can be used.

### (5) Method for regenerating SAM using cell

The method for regenerating S-adenosylmethionine according to the present invention can be carried out using a cell (in a cell) engineered so as to enhance the activity or expression of each enzyme described in the above section (4). Specifically, the present invention provides a method for regenerating SAM using a cell.

In the present invention, the method for regenerating SAM using a cell can comprise, for example, the steps of:
(a) preparing and culturing a cell having a formic acid fixation pathway; and
(b) adding formic acid or a salt thereof to a culture solution of the cell obtained in the step (a), followed by further culture.

In the step (a), the "cell having a formic acid fixation pathway" can be prepared, for example, by introducing DNAs encoding FtfL, Fch, and MtdA to a cell by use of a known transfection method. Specifically, in the present invention, the "cell having a formic acid fixation pathway" is, for example, a cell comprising DNAs encoding FtfL, Fch, and MtdA. Those skilled in the art can appropriately set the culture conditions (a temperature, a time, the composition of a medium, etc.) of the cell depending on the type of the cell.

In the step (b), the "formic acid or salt thereof" is as described in the above section "(1) Formic acid." In the step (b), a methylation target substance (substance to be methylated) can be further added.

The reaction conditions (a temperature, a reaction time, pH, etc.) of the enzyme used in the present invention are as described above.

In the present invention, the method for regenerating SAM using a cell, in the case of using *E. coli* as the cell, can be performed, for example, as follows, though the method is not limited thereto.

First, the host cell *E. coli* is transfected with a DNA encoding each enzyme to prepare a transformant. The transformant is cultured overnight at approximately 37°C in a known culture medium (e.g., LB medium). Next, the transformant that has proliferated is suspended in a minimum medium (e.g., M9 medium), cultured at approximately 30°C for an appropriate time (e.g., 6 hours), then induced to express the gene using IPTG or the like, and further cultured for an appropriate time (e.g., 16 hours). A transformant with enhanced activity or expression of each enzyme can thereby be prepared. Next, the transformant with enhanced activity or expression of each enzyme and a formic acid-containing solution (reaction buffer) are contacted with each other (e.g., the transformant is suspended in a formic acid-containing solution). A methylation target substance is added thereto, and the transformant is further cultured at an appropriate temperature (e.g., approximately 30°C) for an appropriate time (e.g., approximately 6 to 24 hours) so that methylation reaction occurs to produce a methyl compound while SAM can be regenerated from SAH resulting from the demethylation of SAM.

The formic acid-containing solution can comprise, in addition to the formic acid, for example, a buffer solution, and glucose as a source of ATP supply and may further comprise a divalent metal salt (e.g., magnesium sulfate).

**In** the present invention, those skilled in the art can evaluate whether or not SAM has been produced by use of a known method, for example, a method of measuring methyltransferase activity in a test sample, as described in the above section "(3) Method for regenerating S-adenosylmethionine."

### 2-2. Method for regenerating S-adenosylmethionine using methanol

### (1) Methanol

Methanol, an alcohol, is a compound represented by the chemical formula CH₃OH. **In** the present invention, methanol is converted to formaldehyde.

### (2) S-Adenosylmethionine

S-Adenosylmethionine is as described in the above section "2-1.(2)."

### (3) Method for regenerating S-adenosylmethionine

The "regeneration" of SAM, the evaluation of whether or not SAM has been produced, a control, the evaluation of methyltransferase activity, and the phrase "higher than that of a control" according to the present invention are as described in the above section "2-1.(3)."

The method for regenerating S-adenosylmethionine according to the present invention can comprise, for example, the steps of:
(a) converting methanol to formaldehyde, and converting the formaldehyde to formic acid;
(b) producing 5-methyltetrahydrofolate from the formic acid and tetrahydrofolate;
(c) converting S-adenosylhomocysteine, which is produced by the demethylation of S-adenosylmethionine, to homocysteine; and
(d) producing methionine by transferring a methyl group of the 5-methyltetrahydrofolate to the homocysteine, and converting the methionine to S-adenosylmethionine.

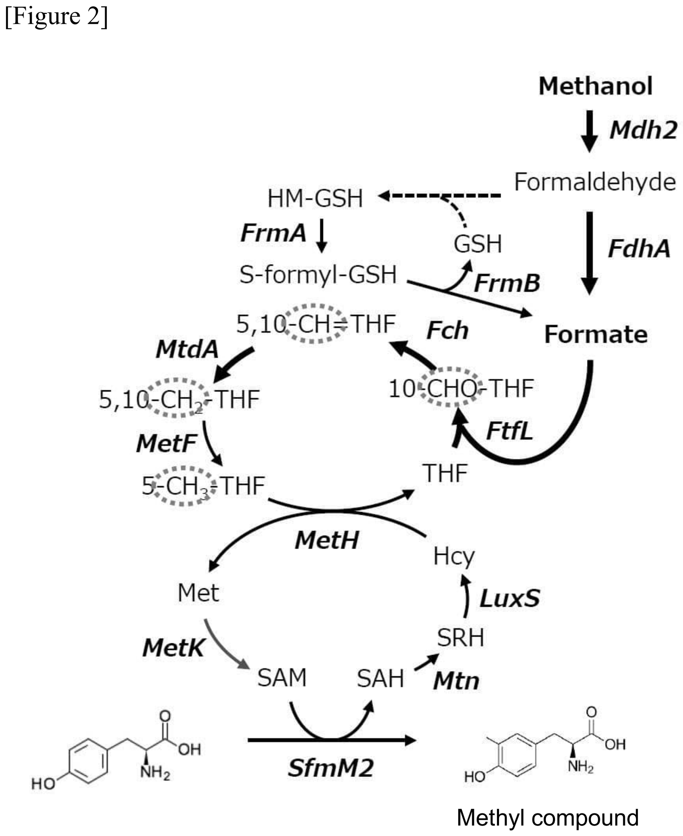

The step (a) is the step of converting methanol to formaldehyde, and producing formic acid from the formaldehyde (Figure 2).

The step (a) can comprise, for example, the steps of: (i) converting methanol to formaldehyde; and (ii) converting the formaldehyde to formic acid.

In the step (i), the conversion of methanol to formaldehyde can be performed in the presence of, for example, methanol dehydrogenase (Medh) and/or methanol oxidase (Mox) or with these enzymes.

The step (ii) includes, for example, the step of (ii-1) directly converting the formaldehyde to formic acid or (ii-2) producing hydroxymethyl glutathione (HM-GSH) through spontaneous reaction of the formaldehyde with glutathione (GSH), converting HM-GSH to S-formylglutathione, and converting the S-formylglutathione to formic acid.

In the step (ii-1), the conversion of the formaldehyde to formic acid can be performed in the presence of, for example, formaldehyde dehydrogenase (FdhA) or with this enzyme.

In the step (ii-2), the production of hydroxymethyl glutathione (HM-GSH) is based on spontaneous reaction of the formaldehyde with glutathione (GSH),
the conversion of HM-GSH to S-formylglutathione can be performed in the presence of, for example, S-hydroxymethyl glutathione dehydrogenase (FrmA) or with this enzyme, and
the conversion of the S-formylglutathione to formic acid can be performed in the presence of, for example, S-formylglutathione hydrolase (FrmB) or with this enzyme.

Specifically, in the step (a), the conversion of the formaldehyde to formic acid can be performed in the presence of, for example, formaldehyde dehydrogenase (FdhA) or with this enzyme.

In another aspect, in the step (a), the conversion of the formaldehyde to formic acid can be performed, following for example spontaneous reaction of formaldehyde with glutathione, in the presence of S-hydroxymethyl glutathione dehydrogenase (FrmA) and S-formylglutathione hydrolase (FrmB) or with these enzymes.

The step (b) is the step of producing 5-methyltetrahydrofolate (5-CH₃-THF) from the formic acid and tetrahydrofolate (THF).

The step (b) can comprise, for example, the steps of: (i) producing 10-formyltetrahydrofolate (10-CHO-THF) from the formic acid and tetrahydrofolate (THF); (ii) producing 5,10-methenyl-tetrahydrofolate (5,10-CH=THF) from 10-CHO-THF; (iii) producing 5,10-methylene-tetrahydrofolate (5,10-CH₂-THF) from 5,10-CH=THF; and (iv) producing 5-methyltetrahydrofolate (5-CH₃-THF) from 5,10-CH₂-THF.

In the step (i), the production of 10-CHO-THF from the formic acid and tetrahydrofolate (THF) can be performed in the presence of, for example, formate-tetrahydrofolate ligase (FtfL) or with this enzyme.

In the step (ii), the production of 5,10-CH=THF from 10-CHO-THF can be performed in the presence of, for example, methenyl-tetrahydrofolate cyclohydrolase (Fch) or with this enzyme.

In the step (iii), the production of 5,10-CH₂-THF from 5,10-CH=THF can be performed in the presence of, for example, methylene-tetrahydrofolate dehydrogenase (MtdA) or with this enzyme.

In the step (iv), the production of 5-CH₃-THF from 5,10-CH₂-THF can be performed in the presence of, for example, 5,10-methylene-tetrahydrofolate reductase (MetF) or with this enzyme.

Specifically, in the step (b), the production of 5-methyltetrahydrofolate from formic acid and tetrahydrofolate can be performed in the presence of, for example, formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF) or with these enzymes.

The step (c) is the step of converting S-adenosylhomocysteine (SAH) produced by the demethylation of S-adenosylmethionine (SAM) to homocysteine (Hcy) (Figure 2).

The step (c) can comprise, for example, the steps of: (i) converting S-adenosylhomocysteine (SAH) to S-ribosylhomocysteine (SRH); and (ii) converting SRH to homocysteine (Hcy).

In the step (i), the conversion of S-adenosylhomocysteine (SAH) to S-ribosylhomocysteine (SRH) can be performed in the presence of, for example, S-adenosylhomocysteine nucleosidase (Mtn) or with this enzyme.

In the step (ii), the conversion of SRH to homocysteine (Hcy) can be performed in the presence of, for example, S-ribosylhomocysteine lyase (LuxS) or with this enzyme.

Specifically, in the step (c), the conversion of S-adenosylhomocysteine to homocysteine can be performed in the presence of S-adenosylhomocysteine nucleosidase (Mtn) and S-ribosylhomocysteine lyase (LuxS) or with these enzymes.

In another aspect, the step (c) can comprise, for example, the step of (iii) directly (without conversion to S-ribosylhomocysteine (SRH)) converting S-adenosylhomocysteine (SAH) to homocysteine (Hcy).

In the step (iii), the conversion of S-adenosylhomocysteine to homocysteine can be performed in the presence of S-adenosylhomocysteine hydrolase (SahH) or with this enzyme.

In the step (c), the demethylation of S-adenosylmethionine (SAM) can be performed in the presence of, for example, S-adenosylmethionine-dependent methyltransferase or with this enzyme.

The S-adenosylmethionine-dependent methyltransferase is as described in the above section "2-1(3)."

The step (d) is the step of producing methionine by transferring a methyl group of the 5-methyltetrahydrofolate produced in the step (b) to the homocysteine (Hcy) obtained by the conversion of S-adenosylhomocysteine (SAH) in the step (c), and converting the produced methionine to S-adenosylmethionine (SAM). SAM is regenerated by this step.

The step (d) can comprise, for example, the steps of: (i) producing methionine by transferring a methyl group of the 5-methyltetrahydrofolate produced in the step (b) to the homocysteine (Hcy) obtained by the conversion of S-adenosylhomocysteine (SAH) in the step (c); and (ii) converting the produced methionine to S-adenosylmethionine (SAM).

In the step (i), the production of methionine from the homocysteine can be performed in the presence of, for example, methionine synthase (MetH or MetE) or with this enzyme.

In the step (ii), the conversion of the methionine to S-adenosylmethionine can be performed in the presence of, for example, methionine adenosyltransferase (MetK) or with this enzyme.

Specifically, the production of methionine from the homocysteine can be performed in the presence of methionine synthase (MetH or MetE) or with this enzyme, and the conversion of the methionine to S-adenosylmethionine can be performed in the presence of methionine adenosyltransferase (MetK) or with this enzyme. The methionine synthase may be any of a cobalamin-dependent form (MetH) and a cobalamin-independent form (MetE).

In the step (ii), the conversion of the methionine to S-adenosylmethionine requires adenosine triphosphate (ATP). This ATP can be synthesized by a step comprising the reaction of at least one or more enzymes selected from the group consisting of ribose-phosphate diphosphokinase (Prs), adenine phosphoribosyltransferase (Apt), and adenosine kinase (ADK).

Ribose-phosphate diphosphokinase (Prs) can synthesize phosphoribosyl pyrophosphate (PRPP) from ribose-5-phosphate (R5P), a metabolite of the pentose phosphate pathway. Adenine phosphoribosyltransferase (Apt) can synthesize adenosine monophosphate (AMP) from PRPP and adenine. Adenosine kinase (ADK) can synthesize adenosine monophosphate (AMP) from adenosine. AMP can be further phosphorylated and thereby converted to ATP.

Adenine is a metabolite resulting from the reaction of Mtn in the step (b), and adenosine is a metabolite resulting from the reaction of SahH in the step (b). These metabolites are utilized as substrates for the synthesis of ATP so that ATP necessary for the synthesis of SAM can be efficiently supplied.

These enzymes for use in the method of the present invention, in the case of using a cell in the regeneration of SAM, may be, for example, endogenous enzymes of the cell or may be expressed by introducing foreign DNAs encoding the enzymes to the cell.

The reaction conditions (a temperature, a reaction time, pH, etc.) of each enzyme in the method of the present invention are the same as those described in the above section "2-1(3)".

In another aspect, the method for regenerating S-adenosylmethionine according to the present invention can comprise, for example, the steps of:
(a) converting methanol to formaldehyde;
(b) producing 5-methyltetrahydrofolate from the formaldehyde and tetrahydrofolate;
(c) converting S-adenosylhomocysteine, which is produced by the demethylation of S-adenosylmethionine, to homocysteine; and
(d) producing methionine by transferring a methyl group of the 5-methyltetrahydrofolate to the homocysteine, and converting the methionine to S-adenosylmethionine.

The step (a) is the step of converting methanol to formaldehyde.

In the step (a), the conversion of methanol to formaldehyde can be performed in the presence of, for example, methanol dehydrogenase (Medh) and/or methanol oxidase (Mox) or with these enzymes.

The step (b) is the step of producing 5-methyltetrahydrofolate (5-CH₃-THF) from the formaldehyde and tetrahydrofolate.

The step (b) can comprise, for example, the steps of: (i) producing 5,10-methylene-tetrahydrofolate (5,10-CH₂-THF) from the formaldehyde and tetrahydrofolate; and (ii) producing 5-methyltetrahydrofolate (5-CH₃-THF) from 5,10-CH₂-THF.

In the step (i), the production of 5,10-CH₂-THF from the formaldehyde and tetrahydrofolate can be performed through spontaneous reaction of the formaldehyde with tetrahydrofolate.

In the step (ii), the production of 5-CH₃-THF from 5,10-CH₂-THF, for example, 5,10-methylene-tetrahydrofolate reductase (MetF) or with this enzyme.

Specifically, in the step (b), the production of 5-CH₃-THF from the formaldehyde and tetrahydrofolate can be performed in the presence of, for example, spontaneous reaction of the formaldehyde with tetrahydrofolate and in the presence of 5,10-methylene-tetrahydrofolate reductase (MetF) or with these enzymes.

Those skilled in the art can perform each step of the present invention in a cell-free system by a known approach (Microbial Cell Factories, Volume 11, Article number 120, September 2012, Journal of Bioscience and Bioengineering, Volume 129, Issue 3, March 2020, Pages 269-275).

The steps (c) and (d) are as described above.

### (4) Cell

The type of the cell that can be used in the method for regenerating S-adenosylmethionine according to the present invention is as described in the above section "2-1(4).".

In the present invention, a cell engineered so as to enhance the activity or expression of S-adenosylmethionine-dependent methyltransferase can be used.

In the present invention, a cell engineered so as to enhance the activity or expression of formate-tetrahydrofolate ligase (FtfL) and SAM-dependent methyltransferase can also be used.

In the present invention, a cell further engineered so as to enhance the activity or expression of methanol dehydrogenase (Medh) and/or methanol oxidase (Mox) in addition to FtfL and SAM-dependent methyltransferase can be used.

In the present invention, a cell engineered so as to enhance the activity or expression of
(i) Medh and/or Mox, and/or
(ii) at least one or more enzymes selected from the group consisting of formaldehyde dehydrogenase (FdhA), S-hydroxymethyl glutathione dehydrogenase (FrmA), and S-formylglutathione hydrolase (FrmB) (e.g., only FdhA; FrmA and FrmB; or FdhA, FrmA and FrmB)
in addition to FtfL and SAM-dependent methyltransferase can be used.

In the present invention, a cell engineered so as to enhance the activity or expression of at least one or more enzymes selected from the group consisting of the following enzymes (i) to (iii):
(i) Medh and/or Mox,
(ii) at least one or more enzymes selected from the group consisting of FdhA, FrmA, and FrmB (e.g., only FdhA; FrmA and FrmB; or FdhA, FrmA and FrmB), and
(iii) methenyl-tetrahydrofolate cyclohydrolase (Fch) and/or methylene-tetrahydrofolate dehydrogenase (MtdA)
in addition to FtfL and SAM-dependent methyltransferase can be used.

In the present invention, a cell engineered so as to enhance the activity or expression of at least one or more enzymes selected from the group consisting of the following enzymes (i) to (iv):
(i) Medh and/or Mox,
(ii) at least one or more enzymes selected from the group consisting of FdhA, FrmA, and FrmB (e.g., only FdhA; FrmA and FrmB; or FdhA, FrmA and FrmB),
(iii) Fch and/or MtdA, and
(iv) at least one or more enzymes selected from the group consisting of S-adenosylhomocysteine nucleosidase (Mtn), S-ribosylhomocysteine lyase (LuxS), and S-adenosylhomocysteine hydrolase (SahH) (e.g., only SahH, or Mtn and Lux)
in addition to FtfL and SAM-dependent methyltransferase can be used.

In the present invention, a cell engineered so as to enhance the activity or expression of at least one or more enzymes selected from the group consisting of the following enzymes (i) to (v):
(i) Medh and/or Mox,
(ii) at least one or more enzymes selected from the group consisting of FdhA, FrmA, and FrmB (e.g., only FdhA; FrmA and FrmB; or FdhA, FrmA and FrmB),
(iii) Fch and/or MtdA,
(iv) at least one or more enzymes selected from the group consisting of Mtn, LuxS, and SahH (e.g., only SahH, or Mtn and Lux), and
(v) at least one or more enzymes selected from the group consisting of Prs, Apt, and ADK (e.g., Prs and Apt or only ADK)
in addition to FtfL and SAM-dependent methyltransferase can be used.

In the present invention, a cell engineered so as to enhance the activity or expression of at least one or more enzymes selected from the group consisting of the following enzymes (i) to (vi):
(i) Medh and/or Mox,
(ii) at least one or more enzymes selected from the group consisting of FdhA, FrmA and FrmB (e.g., only FdhA; FrmA and FrmB; or FdhA, FrmA and FrmB),
(iii) Fch and/or MtdA,
(iv) at least one or more enzymes selected from the group consisting of Mtn, LuxS, and SahH (e.g., only SahH, or Mtn and Lux),
(v) at least one or more enzymes selected from the group consisting of Prs, Apt, and ADK (e.g., Prs and Apt or only ADK), and
(vi) at least one or more enzymes selected from the group consisting of 5,10-methylene-tetrahydrofolate reductase (MetF), methionine synthase (MetH or MetE), and methionine adenosyltransferase (MetK)
in addition to FtfL and SAM-dependent methyltransferase can be used.

In the present invention, a cell engineered so as to enhance the activity or expression of at least one or more enzymes selected from the group consisting of the following enzymes (i) to (iv):
(i) Medh and/or Mox,
(ii) at least one or more enzymes selected from the group consisting of FdhA, FrmA, and FrmB (e.g., only FdhA; FrmA and FrmB; or FdhA, FrmA and FrmB),
(iii) Fch and/or MtdA, and
(iv) at least one or more enzymes selected from the group consisting of MetF, methionine synthase (MetH or MetE), and MetK
in addition to FtfL and SAM-dependent methyltransferase can be used.

In another aspect, in the present invention, a cell further engineered so as to enhance the activity or expression of Medh and/or Mox in addition to SAM-dependent methyltransferase can be used.

In the present invention, a cell engineered so as to enhance the activity or expression of
(i) Medh and/or Mox, and/or
(ii) at least one or more enzymes selected from the group consisting of Mtn, LuxS, and SahH (e.g., only SahH, or Mtn and Lux)
in addition to SAM-dependent methyltransferase can be used.

In the present invention, a cell engineered so as to enhance the activity or expression of at least one or more enzymes selected from the group consisting of the following enzymes (i) to (iii):
(i) Medh and/or Mox,
(ii) at least one or more enzymes selected from the group consisting of Mtn, LuxS, and SahH (e.g., only SahH, or Mtn and Lux), and
(iii) at least one or more enzymes selected from the group consisting of Prs, Apt, and ADK (e.g., Prs and Apt or only ADK)
in addition to SAM-dependent methyltransferase can be used.

In the present invention, a cell engineered so as to enhance the activity or expression of at least one or more enzymes selected from the group consisting of the following enzymes (i) to (iv):
(i) Medh and/or Mox,
(ii) at least one or more enzymes selected from the group consisting of Mtn, LuxS, and SahH (e.g., only SahH, or Mtn and Lux),
(iii) at least one or more enzymes selected from the group consisting of Prs, Apt, and ADK (e.g., Prs and Apt or only ADK), and
(iv) at least one or more enzymes selected from the group consisting of MetF, methionine synthase (MetH or MetE), and MetK
in addition to SAM-dependent methyltransferase can be used.

As used herein, the phrase "engineered so as to enhance the activity or expression" of an enzyme and the cell engineered so as to enhance the activity or expression of SAM-dependent methyltransferase are as described in the above section "2-1(4)."

Examples of the cell engineered so as to enhance the activity or expression of each of FtfL, Medh, Mox, FdhA, FrmA, FrmB, Fch, MtdA, Mtn, LuxS, SahH, Prs, Apt, ADK, MetF, MetH, MetE, and MetK include cells comprising a DNA encoding each of FtfL, Medh, Mox, FdhA, FrmA, FrmB, Fch, MtdA, Mtn, LuxS, SahH, Prs, Apt, ADK, MetF, MetH, MetE, and MetK.

In the present invention, the "cell engineered" and the cell comprising a DNA encoding each enzyme can also be referred to as a "transformant."

The DNA encoding each of SAM-dependent methyltransferase, FtfL, Medh, Mox, FdhA, FrmA, FrmB, Fch, MtdA, Mtn, LuxS, SahH, Prs, Apt, ADK, MetF, MetH, MetE, and MetK can be prepared by obtaining information on the nucleotide sequence thereof from a known database, for example, NCBI (https://www.ncbi.nlm.nih.gov/nucleotide/) and using a known genetic engineering approach.

The vector that can be used in the present invention is as described in the above section "2-1(4)."

In the present invention, the vector can comprise DNAs encoding one or more enzymes. For example, one vector may comprise DNAs encoding FtfL, Fch, and MtdA, DNAs encoding MetF, MetH, and MetK, DNAs encoding Mtn and LuxS, DNAs encoding DNA, MetF, MetH, MetK, Mtn, and LuxS, DNAs encoding Prs and Apt, DNAs encoding Prs, Apt, and ADK, or a DNA encoding ADK.

In the present invention, for example, a reaction system that converts methanol to formaldehyde can be introduced to a cell by introducing a DNA encoding Medh and/or Mox to the cell. Examples of the DNA encoding Medh include the *Cupriavidus necator-derived* mdh2 gene. It is also known that a mutation can be introduced to this mdh2 gene and thereby engineer the gene so as to improve the substrate specificity and activity of Medh against methanol (Appl Microbiol Biotechnol. 2016 Jun; 100 (11): 4969-83.). Such a mutant gene can also be used.

In the present invention, a reaction system with enhanced reaction that converts the formaldehyde obtained by the conversion of methanol to formic acid can be introduced to a cell by introducing, for example, (i) a DNA encoding FdhA and/or (ii) DNAs encoding FrmA and FrmB in addition to the DNA encoding Medh and/or Mox to the cell.

In the present invention, a formic acid fixation pathway that produces 5-methyltetrahydrofolate from the formic acid obtained by the conversion of the formaldehyde can be further introduced to a cell by introducing, for example, (iii) DNAs encoding FtfL, Fch, and MtdA in addition to, for example, the DNA encoding Medh and/or Mox and (i) the DNA encoding FdhA and/or (ii) the DNAs encoding FrmA and FrmB to the cell. Specifically, in the present invention, a cell having a formic acid fixation pathway can be used.

### (5) Method for regenerating SAM using cell

The method for regenerating S-adenosylmethionine according to the present invention can be carried out using a cell (in a cell) engineered so as to enhance the activity or expression of each enzyme described in the above section (4). Specifically, the present invention provides a method for regenerating SAM using a cell.

In the present invention, the method for regenerating SAM using a cell can comprise, for example, the steps of:
(a) preparing and culturing a cell comprising (i) a DNA encoding Medh and/or Mox, (ii-1) a DNA encoding FdhA and/or (ii-2) DNAs encoding FrmA and FrmB, and (iii) DNAs encoding FtfL, Fch, and MtdA; and
(b) adding methanol to a culture solution of the cell obtained in step (a), followed by further culture.

Those skilled in the art can appropriately set the culture conditions (a temperature, a time, the composition of a medium, etc.) of the cell in the method depending on the type of the cell. The reaction conditions (a temperature, a reaction time, pH, etc.) of each enzyme used in the present invention are as described above.

In the present invention, the method for regenerating SAM using a cell, in the case of using *E. coli* as the cell, can be performed, for example, as follows, though the method is not limited thereto.

First, the host cell *E. coli* is transfected with a DNA encoding each enzyme to prepare a transformant. The transformant is cultured overnight at approximately 37°C in a known culture medium (e.g., LB medium). Next, the transformant that has proliferated is suspended in a minimum medium (e.g., M9 medium), cultured at approximately 30°C for an appropriate time (e.g., 6 hours), then induced to express the gene using IPTG or the like, and further cultured for an appropriate time (e.g., 16 hours). A transformant with enhanced activity or expression of each enzyme can thereby be prepared.

Next, the transformant with enhanced activity or expression of each enzyme and a methanol-containing solution (reaction buffer) are contacted with each other (e.g., the transformant is suspended in a methanol-containing solution). A methylation target substance is added thereto, and the transformant is further cultured at an appropriate temperature (e.g., approximately 30°C) for an appropriate time (e.g., approximately 6 to 24 hours) so that methylation reaction occurs to produce a methyl compound while SAM can be regenerated from SAH resulting from the demethylation of SAM.

The methanol-containing solution can comprise, in addition to the methanol, for example, a buffer solution, and glucose as a source of ATP supply and may further comprise a divalent metal salt (e.g., magnesium sulfate).

In the present invention, those skilled in the art can evaluate whether or not SAM has been produced by use of a known method, for example, a method of measuring methyltransferase activity in a test sample, as described in the above section "(3) Method for regenerating S-adenosylmethionine."

### 3. Method for producing methyl compound

### (1) Methyl compound

As used herein, the "methyl compound" means a compound having a methyl group. Specifically, the methyl compound according to the present invention is, for example, a compound having a methyl group derived from SAM (transferred from SAM). **In** the present invention, the methyl compound results from, for example, the transfer of a methyl group of SAM to a methylation target substance by the action of SAM-dependent methyltransferase. Specifically, the methyl compound according to the present invention is not limited as long as the methyl compound has been produced or can be produced in the presence of SAM. Examples of the methyl compound that has been produced or can be produced in the presence of SAM include derivatives of aromatic amino acids. Examples of the aromatic amino acid include tyrosine, histidine, tryptophan, and phenylalanine. Examples of the methyl compound which is a derivative of an aromatic amino acid include, but are not limited to, 3-methyltyrosine, hercynine, ergothioneine, melatonin, vanillic acid, vanillin, isovanillic acid, ferulic acid, isoferulic acid, pinostilbene, pterostilbene, reticuline, thebaine, codeine, magnoflorine, berberine, ononitol, and pinitol.

**In** one aspect of the present invention, the methyl compound may be at least one selected from the group consisting of, for example, 3-methyltyrosine, hercynine, ergothioneine, melatonin, reticuline, vanillic acid, vanillin, isovanillic acid, ferulic acid, isoferulic acid, pinostilbene, pterostilbene, thebaine, codeine, magnoflorine, berberine, ononitol, and pinitol.

**In** an alternative aspect of the present invention, the methyl compound may be at least one selected from the group consisting of, for example, 3-methyltyrosine, hercynine, ergothioneine, melatonin, and reticuline.

**In** an alternative aspect of the present invention, the methyl compound may be at least one selected from the group consisting of, for example, 3-methyltyrosine, hercynine, ergothioneine, and melatonin.

### (2-1) Method for producing methyl compound - 1

The method for producing a methyl compound according to the present invention can comprise, for example, the following steps:
in the presence of formic acid or a salt thereof,
(a) producing 5-methyltetrahydrofolate from formic acid or a salt thereof and tetrahydrofolate;
(b) converting S-adenosylhomocysteine, which is produced by the demethylation of S-adenosylmethionine, to homocysteine;
(c) producing methionine by transferring a methyl group of the 5-methyltetrahydrofolate to the homocysteine, and converting the methionine to S-adenosylmethionine; and
(d) performing methylation reaction with S-adenosylmethionine as a methyl group donor to produce a methyl compound.

The steps (a) to (c) are as described in the above section "(3) Method for regenerating S-adenosylmethionine."

The step (d) is the step of performing methylation reaction with the S-adenosylmethionine (SAM) as a methyl group donor to produce a methyl compound. **In** this context, the "S-adenosylmethionine" in the step (d) may be the S-adenosylmethionine produced in the step (c) or S-adenosylmethionine produced by another method.

The step (d) can comprise, for example, the step of producing a methyl compound from a methylation target substance. The production of a methyl compound from a methylation target substance can be performed, for example, by transferring a methyl group of S-adenosylmethionine (SAM) to a methylation target substance in the presence of SAM-dependent methyltransferase or with this enzyme.

The method for producing a methyl compound according to the present invention can further comprise the step of (e) converting methanol to formaldehyde, and converting the formaldehyde to formic acid.

In the step (e), the conversion of methanol to formaldehyde may be performed in the presence of methanol dehydrogenase (Medh) and/or methanol oxidase (Mox).

In the step (e), the conversion of the formaldehyde to formic acid may be performed in the presence of formaldehyde dehydrogenase (FdhA).

In the step (e), the conversion of the formaldehyde to formic acid may be performed in the presence of spontaneous reaction of formaldehyde with glutathione, and in the presence of S-hydroxymethyl glutathione dehydrogenase (FrmA) and S-formylglutathione hydrolase (FrmB).

The method for producing a methyl compound according to the present invention can be performed in a cell-free system by a known approach using each enzyme described above.

The method for producing a methyl compound according to the present invention can be carried out using a cell (in a cell) engineered so as to enhance the activity or expression of each enzyme described in the above section "2.(4)." Specifically, the present invention provides a method for producing a methyl compound using a cell.

In the present invention, the method for producing a methyl compound using a cell can comprise, for example, the steps of:
(a) preparing and culturing a cell having a formic acid fixation pathway; and
(b) adding formic acid or a salt thereof and a methylation target substance to a culture solution of the cell obtained in the step (a), followed by further culture, and recovering a methyl compound.

In the step (a), the "cell having a formic acid fixation pathway" is as described in the above section "2.(5) Method for regenerating SAM using cell."

In the step (b), the "formic acid or salt thereof" is as described in the above section "2.(1) Formic acid." The step (b) can further comprise the step of purifying a produced methyl compound.

Those skilled in the art can recover and purify the methyl compound produced in the step (b) by a known method suitable for the physical properties of the methyl compound, for example, treatment such as distillation, membrane dehydration, salting-out using an ionexchange resin or the like, crystallization, or column chromatography.

### (2-2) Method for producing methyl compound - 2

In another aspect, the method for producing a methyl compound according to the present invention can comprise, for example, the steps of:
(a) converting methanol to formaldehyde, and converting the formaldehyde to formic acid;
(b) producing 5-methyltetrahydrofolate from the formic acid and tetrahydrofolate;
(c) converting S-adenosylhomocysteine, which is produced by the demethylation of S-adenosylmethionine, to homocysteine;
(d) producing methionine by transferring a methyl group of the 5-methyltetrahydrofolate to the homocysteine, and converting the methionine to S-adenosylmethionine; and
(e) performing methylation reaction with the S-adenosylmethionine produced in the step (d) as a methyl group donor to produce a methyl compound.

The steps (a) to (d) are as described in the above section "(3) Method for regenerating S-adenosylmethionine. "

The step (e) is the step of performing methylation reaction with the produced S-adenosylmethionine (SAM) as a methyl group donor to produce a methyl compound.

The step (e) can comprise, for example, the step of producing a methyl compound from a methylation target substance. The production of a methyl compound from a methylation target substance can be performed, for example, by transferring a methyl group of S-adenosylmethionine (SAM) to a methylation target substance in the presence of SAM-dependent methyltransferase or with this enzyme.

The method for producing a methyl compound according to the present invention can be carried out using a cell (in a cell) engineered so as to enhance the activity or expression of each enzyme described in the above section "2.(4)." Specifically, the present invention provides a method for producing a methyl compound using a cell.

In the present invention, the method for producing a methyl compound using a cell can comprise, for example, the steps of:
(a) preparing and culturing a cell comprising (i) a DNA encoding Medh and/or Mox, (ii-1) a DNA encoding FdhA and/or (ii-2) DNAs encoding FrmA and FrmB, and (iii) DNAs encoding FtfL, Fch, and MtdA; and
(b) adding methanol and a methylation target substance to a culture solution of the cell obtained in the step (a), followed by further culture, and recovering a methyl compound.

The step (b) can further comprise the step of purifying the produced methyl compound.

Those skilled in the art can recover and purify the methyl compound produced in the step (b) by a known method suitable for the physical properties of the methyl compound, for example, treatment such as distillation, membrane dehydration, salting-out using an ionexchange resin or the like, crystallization, or column chromatography.

In the present invention, the method for producing a methyl compound using a cell, in the case of using *E. coli* as the cell, can be performed, for example, as follows, though the method is not limited thereto.

First, the host cell *E. coli* is transfected with a DNA encoding each enzyme to prepare a transformant. The transformant is cultured overnight at approximately 37°C in a known culture medium (e.g., LB medium). Next, the transformant that has proliferated is suspended in a minimum medium (e.g., M9 medium), cultured at approximately 30°C for an appropriate time (e.g., 6 hours), then induced to express the gene using IPTG or the like, and further cultured for an appropriate time (e.g., 16 hours). A transformant with enhanced activity or expression of each enzyme can thereby be prepared.

Next, the transformant with enhanced activity or expression of each enzyme and a formic acid-containing solution or a methanol-containing solution (reaction buffer) are contacted with each other (e.g., the transformant is suspended in a formic acid-containing solution or a methanol-containing solution). A methylation target substance is added thereto, and the transformant is further cultured at an appropriate temperature (e.g., approximately 30°C) for an appropriate time (e.g., approximately 6 to 24 hours) so that methylation reaction occurs to produce a methyl compound.

The formic acid-containing solution and the methanol-containing solution can each comprise, in addition to the formic acid or the methanol, for example, a buffer solution, and glucose as a source of ATP supply and may further comprise a divalent metal salt (e.g., magnesium sulfate).

### 4. Method for producing organic compound

In the present invention, an organic compound can be produced using a methyl compound produced by the method for regenerating SAM or the method for producing a methyl compound according to the present invention as an intermediate. Specifically, the present invention provides a method for producing an organic compound, comprising the step of producing the organic compound via a methyl compound produced by the method for regenerating SAM or the method for producing a methyl compound according to the present invention as an intermediate.

As used herein, the organic compound means a compound produced via a methyl compound as an intermediate. Examples of the methyl compound for use as an intermediate include compounds produced by the method for regenerating SAM or the method for producing a methyl compound according to the present invention.

Those skilled in the art can produce an organic compound via the methyl compound as an intermediate on the basis of a known approach. Those skilled in the art can also appropriately set the production conditions thereof.

Examples of the organic compound that can be produced via the methyl compound as an intermediate include o-cresol, 2,6-xylenol, cumic acid, terephthalic acid, and ergothioneine. Tyrosine is methylated by the method for regenerating SAM or the method for producing a methyl compound according to the present invention to produce 3-methyltyrosine or 3,5-dimethyltyrosine, and such a methyl compound can then be converted to o-cresol or 2,6-xylenol using lyase that recognizes each compound as a substrate. Perillic acid is methylated by the method for regenerating SAM or the method for producing a methyl compound according to the present invention to produce methyl perillate, which can then be converted to cumic acid and further to terephthalic acid through known chemical synthesis reaction (ChemistryOpen. 2018 Feb; 7 (2): 201-203). L-Histidine is methylated by the method for regenerating SAM or the method for producing a methyl compound according to the present invention, and ergothioneine can be produced as an organic compound via hercynine as an intermediate.

Hereinafter, the present invention will be described in detail with reference to Examples. However, the present invention is not limited to these Examples. In the following Examples, the term "enhancement" or "enhanced" means that the activity or expression of an enzyme was enhanced.

### [Examples]

### [Example 1]

The expression of sfmM2 gene, ftfL gene, fch gene, and mtdA gene was enhanced in *E*. *coli* HMS174(DE3) strain as follows.

### (A) Construction of SfmM2 enhancement plasmid

A DNA having a nucleotide sequence (sequence with recognition sequences of restriction enzymes Nde I and Bgl II added to the 5'- and 3'-terminal sides, respectively) comprising *Streptomyces lavendulae*-derived sfmM2 gene encoding tyrosine 3C-methyltransferase was synthesized (nucleotide sequence of synthetic DNA: SEQ ID NO: 1). The nucleotide sequence of the sfmM2 gene was designed as a codon-optimized sequence for efficient expression in *E. coli.* The synthesized DNA was inserted to restriction enzyme sites Nde I and Bgl II of an expression vector pET Duet-1 for *E. coli* (Novagen) and linked downstream of T7 promoter. The constructed plasmid was designated as pET_P_{T7}-sfmM2.

### (B) Construction of FtfL, Fch, MtdA enhancement plasmid

A DNA having a nucleotide sequence (sequence with recognition sequences of restriction enzymes Nde I and Bgl II added to the 5'- and 3'-terminal sides, respectively) comprising *Methylobacterium extorquens*-derived ftfL gene, fch gene, and mtdA gene was synthesized (nucleotide sequence of synthetic DNA: SEQ ID NO: 2). The nucleotide sequence comprising the ftfL gene, the fch gene, and the mtdA gene was designed as a codon-optimized sequence for efficient expression in *E. coli.* The synthesized DNA was inserted to restriction enzyme sites Nde I and Bgl II of an expression vector pCOLA Duet-1 for *E. coli* (Novagen) and linked downstream of T7 promoter. The constructed plasmid was designated as pCOLA_P_{T7}-ftfL-fch-mtdA.

### (C) Preparation of SfmM2, FtfL, Fch, MtdA-enhanced strain

*E. coli* HMS174(DE3) strain (Novagen) was transformed with the plasmid pET_P_{T7}-sfmM2 constructed in Example 1(A), the plasmid pCOLA_P_{T7}-ftfL-fch-mtdA constructed in Example 1(B), and pCDFDuet-1 (Novagen). The obtained strain was designated as HMS174(DE3)/pET_P_{T7}-sfmM2/pCOLA_P_{T7}-ftfL-fch-mtdA.

### [Example 2]

The expression of sfmM2 gene, luxS gene, ftfL gene, fch gene, and mtdA gene was enhanced in *E. coli* HMS174(DE3) strain as follows.

### (A) Construction of Mtn, LuxS enhancement plasmid

A DNA having a nucleotide sequence (sequence with recognition sequences of restriction enzymes Nde I and Bgl II added to the 5'- and 3'-terminal sides, respectively) comprising *Escherichia coli*-derived mtn gene and luxS gene was synthesized (nucleotide sequence of synthetic DNA: SEQ ID NO: 3). The synthesized DNA was inserted to restriction enzyme sites Nde I and Bgl II of an expression vector pCDF Duet-1 for *E. coli* (Novagen) and linked downstream of T7 promoter. The constructed plasmid was designated as pCDF_P_{T7}-mtn-luxS.

### (B) Construction of LuxS enhancement plasmid

The plasmid pCDF_P_{T7}-mtn-luxS constructed in Example 2(A) was treated with a restriction enzyme Nde I, separated by agarose gel electrophoresis, then recovered from a gel, and purified to obtain a DNA fragment from which the mtn gene region was removed. Both ends of the obtained DNA fragment were self-ligated for linking. The constructed plasmid was designated as pCDF_P_{T7}-luxS.

### (C) Preparation of SfmM2, LuxS, FtfL, Fch, MtdA-enhanced strain

*E. coli* HMS174(DE3) strain (Novagen) was transformed with the plasmid pET_P_{T7}-sfmM2 constructed in Example 1(A), the plasmid pCOLA_P_{T7}-ftfL-fch-mtdA constructed in Example 1(B), and the plasmid pCDF_P_{T7}-luxS constructed in Example 2(B). The obtained strain was designated as HMS174(DE3)/pET_P_{T7}-sfmM2/pCDF_P_{T7}-luxS/pCOLA_P_{T7}-ftfL-fch-mtdA.

### [Example 3]

The expression of sfmM2 gene, mtn gene, luxS gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCOLA_P_{T7}-ftfL-fch-mtdA constructed in Example 1(B), and the plasmid pCDF_P_{T7}-mtn-luxS constructed in Example 2(A). The obtained strain was designated as HMS174(DE3)/pET_P_{T7}-sfmM2/pCDF_P_{T7}-mtn-luxS/pCOLA_P_{T7}-ftfL-fch-mtdA.

### [Example 4]

The expression of sfmM2 gene, sahH gene, ftfL gene, fch gene, and mtdA gene was enhanced in *E. coli* HMS174(DE3) strain as follows.

### (A) Construction of SahH enhancement plasmid

A DNA having a nucleotide sequence (sequence with recognition sequences of restriction enzymes Nde I and Bgl II added to the 5'- and 3'-terminal sides, respectively) comprising *Pseudomonas aeruginosa*-derived sahH gene was synthesized (nucleotide sequence of synthetic DNA: SEQ ID NO: 4). The nucleotide sequence of the sahH gene was designed as a codon-optimized sequence for efficient expression in *E. coli.* The synthesized DNA was inserted to restriction enzyme sites Nde I and Bgl II of an expression vector pCDF Duet-1 for *E. coli* (Novagen) and linked downstream of T7 promoter. The constructed plasmid was designated as pCDF_P_{T7}-sahH.

### (B) Preparation of SfmM2, SahH, FtfL, Fch, MtdA-enhanced strain

*E. coli* HMS174(DE3) strain (Novagen) was transformed with the plasmid pET_P_{T7}-sfmM2 constructed in Example 1(A), the plasmid pCOLA_P_{T7}-ftfL-fch-mtdA constructed in Example 1(B), and the plasmid pCDF_P_{T7}-sahH constructed in Example 4(A). The obtained strain was designated as HMS174(DE3)/pET_P_{T7}-sfmM2/pCDF_P_{T7}-sahH/pCOLA_P_{T7}-ftfL-fch-mtdA.

### [Example 5]

The SfmM2, FtfL, Fch, MtdA-enhanced strain prepared in Example 1(C) was evaluated for 3-methyltyrosine production as follows.

HMS174(DE3)/pET_P_{T7}-sfmM2/pCOLA_P_{T7}-ftfL-fch-mtdA prepared in Example 1(C) was cultured overnight in LB medium (16 g/L Bacto-trypton, 10 g/L Yeast extract, 5 g/L NaCl) comprising 50 µg/mL carbenicillin, 50 µg/mL spectinomycin, and 50 µg/mL kanamycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 50 µg/mL kanamycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 10 in a reaction buffer (100 mM Tris-HCl pH 8.0, 20 mM MgSO₄, 20 mM glucose) comprising 20 mM sodium formate, 100 µg/mL carbenicillin, 100 µg/mL spectinomycin, 100 µg/mL kanamycin, and 1 mM IPTG. 1 mL of the cell suspension was added to a 14 mL tube and mixed with 1 mL of a 10 mM L-tyrosine solution (dissolved in 0.1 N hydrochloric acid) serving as a substrate to start methylation reaction. The reaction solution was shaken at 200 rpm at 30°C for 6 hours.

In order to make sure that methylation reaction could be accelerated by efficient regeneration of methyl group carbon of SAM, a test conducted under the same conditions as above except that sodium formate serving as a source of methyl group carbon supply was not added was also carried out, and the obtained results were compared with the results of the test conducted under the sodium formate addition conditions.

20 µL of the obtained reaction solution was mixed with 20 µL of pure water, 80 µL of 1 N hydrochloric acid, and 80 µL of acetonitrile, and a supernatant was collected after centrifugation at 3000 × g for 5 minutes and subjected to LC-MS analysis. The conditions of LC-MS analysis are shown in Table 1.

As a result of measurement, a 3-methyltyrosine concentration of the reaction solution was 0.092 mM under the sodium formate addition conditions, which was increased by 0.030 mM as compared with the sodium formate non-addition conditions.

**[Table 1]**

| | |
|---|---|
| Apparatus | Waters UPLC-MS (SQD2) |
| Column | Acquity UPLC BEH C18 1.7 µm 2.1 x 50 mm |
| Column temperature | 40°C |
| Mobile phase | (A) 0.1% aqueous formic acid solution |
| | (B) Acetonitrile containing 0.1% formic acid |
| Detection | PDA (245-400 nm), MS (ESI +/-) |
| Flow rate | 0.2 mL/min |
| Elution (B%) | Held at 0% for 1 min, elevated to 90% over 4.5 min, held at 90% for 1 min. Lowered to 0% over 0.5 min, and then held at 0% for 3 min. |

### [Example 6]

The SfmM2, LuxS, FtfL, Fch, MtdA-enhanced strain prepared in Example 2(C) was evaluated for 3-methyltyrosine production as follows.

In this Example, the 3-methyltyrosine production was evaluated in the same manner as in Example 5 except that HMS174(DE3)/pET_P_{T7}-sfmM2/pCDF_P_{T7}-luxS/pCOLA_P_{T7}-ftfL-fch-mtdA prepared in Example 2(C) was used.

As a result, a 3-methyltyrosine concentration of the reaction solution was 0.185 mM under the sodium formate addition conditions, which was increased by 0.136 mM as compared with the sodium formate non-addition conditions.

### [Example 7]

The SfmM2, Mtn, LuxS, FtfL, Fch, MtdA-enhanced strain prepared in Example 3 was evaluated for 3-methyltyrosine production as follows.

In this Example, the 3-methyltyrosine production was evaluated in the same manner as in Example 5 except that HMS174(DE3)/pET_P_{T7}-sfmM2/pCDF_P_{T7}-Mtn-luxS/pCOLA_P_{T7}-ftfL-fch-mtdA prepared in Example 3 was used.

As a result, a 3-methyltyrosine concentration of the reaction solution was 0.236 mM under the sodium formate addition conditions, which was increased by 0.128 mM as compared with the sodium formate non-addition conditions.

### [Example 8]

The SfmM2, SahH, FtfL, Fch, MtdA-enhanced strain prepared in Example 4(B) was evaluated for 3-methyltyrosine production as follows.

In this Example, the 3-methyltyrosine production was evaluated in the same manner as in Example 5 except that HMS174(DE3)/pET_P_{T7}-sfmM2/pCDF_P_{T7}-sahH/pCOLA_P_{T7}-ftfL-fch-mtdA prepared in Example 4(B) was used.

As a result, a 3-methyltyrosine concentration of the reaction solution was 0.091 mM under the sodium formate addition conditions, which was increased by 0.024 mM as compared with the sodium formate non-addition conditions.

### [Comparative Example 1]

The expression of sfmM2 gene was enhanced in *E*. *coli* HMS174(DE3) strain as follows.

*E. coli* HMS174(DE3) strain (Novagen) was transformed with the plasmid pET_P_{T7}-sfmM2 constructed in Example 1(A), pCDF Duet-1 (Novagen), and pCOLA Duet-1 (Novagen). The obtained strain was designated as HMS174(DE3)/pET_P_{T7}-sfmM2.

### [Comparative Example 2]

The expression of sfmM2 gene and luxS gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-luxS constructed in Example 2(B), and pCOLA Duet-1 (Novagen). The obtained strain was designated as HMS174(DE3)/pET_P_{T7}-sfmM2/pCDF_P_{T7}-luxS.

### [Comparative Example 3]

The expression of sfmM2 gene, mtn gene, and luxS gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-mtn-luxS constructed in Example 2(A), and pCOLADuet-1 (Novagen). The obtained strain was designated as HMS174(DE3)/pET_P_{T7}-sfmM2/pCDF_P_{T7}-mtn-luxS.

### [Comparative Example 4]

The expression of sfmM2 gene and sahH gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-sahH constructed in Example 4(A), and pCOLA Duet-1 (Novagen). The obtained strain was designated as HMS174(DE3)/pET_P_{T7}-sfmM2/pCDF_P_{T7}-sahH.

### [Comparative Example 5]

The SfmM2-enhanced strain prepared in Comparative Example 1 was evaluated for 3-methyltyrosine production as follows.

In this Comparative Example, the 3-methyltyrosine production was evaluated in the same manner as in Example 5 except that HMS174(DE3)/pET_P_{T7}-sfmM2 prepared in Comparative Example 1 was used.

As a result, a 3-methyltyrosine concentration of the reaction solution was not increased as compared with the sodium formate non-addition conditions.

### [Comparative Example 6]

The SfmM2, LuxS-enhanced strain prepared in Comparative Example 2 was evaluated for 3-methyltyrosine production as follows.

In this Comparative Example, the 3-methyltyrosine production was evaluated in the same manner as in Example 5 except that HMS174(DE3)/pET_P_{T7}-sfmM2/pCDF_P_{T7}-luxS prepared in Comparative Example 2 was used.

As a result, a 3-methyltyrosine concentration of the reaction solution was not increased as compared with the sodium formate non-addition conditions.

### [Comparative Example 7]

The SfmM2, Mtn, LuxS-enhanced strain prepared in Comparative Example 3 was evaluated for 3-methyltyrosine production as follows.

In this Comparative Example, the 3-methyltyrosine production was evaluated in the same manner as in Example 5 except that HMS174(DE3)/pET_P_{T7}-sfmM2/pCDF_P_{T7}-mtn-luxS prepared in Comparative Example 3 was used.

As a result, a 3-methyltyrosine concentration of the reaction solution was not increased as compared with the sodium formate non-addition conditions.

### [Comparative Example 8]

The SfmM2, SahH-enhanced strain prepared in Comparative Example 4 was evaluated for 3-methyltyrosine production as follows.

In this Comparative Example, the 3-methyltyrosine production was evaluated in the same manner as in Example 5 except that HMS174(DE3)/pET_P_{T7}-stmM2/pCDF_P₁₇-sahH prepared in Comparative Example 4 was used.

As a result, a 3-methyltyrosine concentration of the reaction solution was not increased as compared with the sodium formate non-addition conditions.

The results of Examples 5 to 8 were summarized in Table 2. In Examples 5, 6, 7, and 8, the concentrations of accumulated 3-methyltyrosine under sodium formate addition conditions were drastically increased to 1.5 times, 3.8 times, 2.2 times, and 1.4 times, respectively, as compared to those under sodium formate non-addition conditions. On the other hand, Comparative Examples 5, 6, 7, and 8 exhibited no increase in 3-methyltyrosine accumulation under sodium formate addition conditions.

These results demonstrated that the use of formic acid under conditions with enhanced expression of FtfL, Fch, and MtdA allows more efficient regeneration of SAM and can accelerate the methylation reaction and increase the amount of an arbitrary methyl compound produced, as compared with the case of using no formic acid or the case of using formic acid under conditions without enhanced expression of the enzymes.

In Examples 6 and 7, the concentrations of accumulated 3-methyltyrosine under sodium formate addition conditions were increased to 2.0 times and 2.6 times, respectively, as compared to the results of Example 5.

These results demonstrated that the enhancement of the expression of LuxS or the expression of Mtn and LuxS improves SAM regeneration efficiency and can further accelerate the methylation reaction.

Specifically, the method of the present invention was found to efficiently regenerate SAM, to accelerate methylation reaction, and to increase the amount of an arbitrary methyl compound produced, by using formic acid.

**[Table 2]**

| | Microbial strain | Source of methyl group supply | Concentration of accumulated 3-methyltyrosine [mM] | Amount of increase in concentration of accumulated 3-methyltyrosine as compared with formic acid non-addition conditions [mM] | Relative value of concentration of accumulated 3-methyltyrosine as compared with formic acid non-addition conditions |
|---|---|---|---|---|---|
| Example 5 | HMS174 (DE3)/pET_P_{T7}-sfmM2/pCOLA_P_{T7}-ftfL-fch-mtdA | Formic acid | 0.092 | 0.030 | 1.5 |
| Example 6 | HMS174 (DE3)/pET_P_{T7}-sftnM2/pCDF_P_{T7}-1uxS/pCOLA_P_{T7}-ftfL-fch-mtdA | Formic acid | 0.185 | 0.136 | 3.8 |
| Example 7 | HMS174 (DE3)/pET_P_{T7}-sfmM2/pCDF_P_{T7}-mtn-1uxS/pCOLA_P_{T7}-ftfL-fch-mtdA | Formic acid | 0.236 | 0.128 | 2.2 |
| Example 8 | HMS174 (DE3)/pET_P_{T7}-sfmM2/pCDF_P_{T7}-sahH/pCOLA_P_{T7}-ftfL-fch- | Formic acid | 0.091 | 0.024 | 1.4 |
| | mtdA | | | | |

### [Example 9]

The expression of TCMT gene, mtn gene, luxS gene, ftfL gene, fch gene, and mtdA gene was enhanced in *E. coli* HMS174(DE3) strain as follows.

### (A) Construction of TCMT enhancement plasmid

A DNA having a nucleotide sequence (sequence with recognition sequences of restriction enzymes Nde I and Bgl II added to the 5'- and 3'-terminal sides, respectively) comprising *Lonsdalea populi*-derived TCMT gene encoding tyrosine 3C-methyltransferase was synthesized (nucleotide sequence of synthetic DNA: SEQ ID NO: 5). The nucleotide sequence of the TCMT gene was designed as a codon-optimized sequence for efficient expression in *E. coli.* The synthesized DNA was inserted to restriction enzyme sites Nde I and Bgl II of an expression vector pET Duet-1 for *E. coli* (Novagen) and linked downstream of T7 promoter. The constructed plasmid was designated as pET_P_{T7}-TCMT.

### (B) Preparation of TCMT, Mtn, LuxS, FtfL, Fch, MtdA-enhanced strain

*E. coli* HMS174(DE3) strain (Novagen) was transformed with the plasmid pET_P_{T7}-TCMT constructed in Example 9(A), the plasmid pCOLA_P_{T7}-ftfL-fch-mtdA constructed in Example 1(B), and the plasmid pCDF_P_{T7}-mtn-luxS constructed in Example 2(A). The obtained strain was designated as HMS174(DE3)/pET_P_{T7}-TCMT/pCDF_P_{T7}-mtn-luxS/pCOLA_P_{T7}-ftfL-fch-mtdA.

### [Example 10]

The expression of TCMT gene, mtn gene, luxS gene, prs gene, apt gene, ftfL gene, fch gene, and mtdA gene was enhanced in *E. coli* HMS174(DE3) strain as follows.

### (A) Construction of Mtn, LuxS, Prs, Apt enhancement plasmid

A DNA having a nucleotide sequence (sequence with recognition sequences of restriction enzymes Bgl II and Fse I added to the 5'- and 3'-terminal sides, respectively) comprising *Escherichia coli*-derived prs gene and apt gene was synthesized (nucleotide sequence of synthetic DNA: SEQ ID NO: 6). The synthesized DNA was inserted to restriction enzyme sites Bgl II and Fse I of the plasmid pCDF_P_{T7}-mtn-luxS constructed in Example 2(A), and linked downstream of the luxS gene. The constructed plasmid was designated as pCDF_P_{T7}-mtn-luxS-prs-apt.

### (B) Preparation of TCMT, Mtn, LuxS, Prs, Apt, FtfL, Fch, MtdA-enhanced strain

*E. coli* HMS174(DE3) strain (Novagen) was transformed with the plasmid pET_P_{T7}-TCMT constructed in Example 9(A), the plasmid pCOLA_P_{T7}-ftfL-fch-mtdA constructed in Example 1(B), and the plasmid pCDF_P_{T7}-mtn-luxS-prs-apt constructed in Example 10(A). The obtained strain was designated as HMS174(DE3)/pET_P₁₇-TCMT/pCDF_P₁₇-mtn-luxS-prs-apt/pCOLA_P_{T7}-ftfL-fch-mtdA.

### [Example 11]

The expression of TCMT gene, sahH gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-TCMT constructed in Example 9(A), the plasmid pCOLA_P_{T7}-ftfL-fch-mtdA constructed in Example 1(B), and the plasmid pCDF_P_{T7}-sahH constructed in Example 4(A). The obtained strain was designated as HMS174(DE3)/pET_P_{T7}-TCMT/pCDF_P_{T7}-sahH/pCOLA_P_{T7}-ftfL-fch-mtdA.

### [Example 12]

The expression of TCMT gene, sahH gene, ADO1 gene, ftfL gene, fch gene, and mtdA gene was enhanced in *E. coli* HMS174(DE3) strain as follows. The ADO1 gene is a gene encoding adenosine kinase (ADK).

### (A) Construction of SahH-ADO1 enhancement plasmid

A DNA having a nucleotide sequence (sequence with recognition sequences of restriction enzymes Bgl II and Mfe I added to the 5'- and 3'-terminal sides, respectively) comprising *Saccharomyces cerevisiae-*derived ADO1 gene was synthesized (nucleotide sequence of synthetic DNA: SEQ ID NO: 7). The nucleotide sequence of the ADO1 gene was designed as a codon-optimized sequence for efficient expression in *E. coli.* The synthesized DNA was inserted to restriction enzyme sites Bgl II and Mfe I of the plasmid pCDF_P_{T7}-sahH constructed in Example 4(A), and linked downstream of the sahH gene. The constructed plasmid was designated as pCDF_P_{T7}-sahH-ADO1.

### (B) Preparation of TCMT, SahH, ADO1, FtfL, Fch, MtdA-enhanced strain

*E. coli* HMS174(DE3) strain (Novagen) was transformed with the plasmid pET_P_{T7}-TCMT constructed in Example 9(A), the plasmid pCOLA_P_{T7}-ftfL-fch-mtdA constructed in Example 1(B), and the plasmid pCDF_P_{T7}-sahH-ADO1 constructed in Example 12(A). The obtained strain was designated as HMS174(DE3)/pET_P_{T7}-TCMT/pCDF_P_{T7}-sahH-ADO1/pCOLA_P_{T7}-ftfL-fch-mtdA.

### [Example 13]

The TCMT, Mtn, LuxS, FtfL, Fch, MtdA-enhanced strain prepared in Example 9(B) was evaluated for 3-methyltyrosine production as follows.

In this Example, the 3-methyltyrosine production was evaluated in the same manner as in Example 5 except that HMS174(DE3)/pET_P_{T7}-TCMT/pCDF_P_{T7}-mtn-luxS/pCOLA_P_{T7}-ftfL-fch-mtdA prepared in Example 9(B) was used; the suspension in a reaction buffer was performed at OD₆₀₀ = 4; and the methylation reaction was performed by shaking at 200 rpm at 30°C for 24 hours.

As a result, a 3-methyltyrosine concentration of the reaction solution was 0.162 mM under the sodium formate addition conditions, which was increased by 0.093 mM as compared with the sodium formate non-addition conditions.

### [Example 14]

The TCMT, Mtn, LuxS, Prs, Apt, FtfL, Fch, MtdA-enhanced strain prepared in Example 10(B) was evaluated for 3-methyltyrosine production as follows.

In this Example, the 3-methyltyrosine production was evaluated in the same manner as in Example 13 except that HMS174(DE3)/pET_P_{T7}-TCMT/pCDF_P_{T7}-mtn-luxS-prs-apt/pCOLA_P_{T7}-ftfL-fch-mtdA prepared in Example 10(B) was used.

As a result, a 3-methyltyrosine concentration of the reaction solution was 0.187 mM under the sodium formate addition conditions, which was increased by 0.141 mM as compared with the sodium formate non-addition conditions.

### [Example 15]

The TCMT, SahH, FtfL, Fch, MtdA-enhanced strain prepared in Example 11 was evaluated for 3-methyltyrosine production as follows.

In this Example, the 3-methyltyrosine production was evaluated in the same manner as in Example 13 except that HMS174(DE3)/pET_P₁₇-TCMT/pCDF_P₁₇-sahH/pCOLA_P₁₇-ftfL-fch-mtdA prepared in Example 11 was used.

As a result, a 3-methyltyrosine concentration of the reaction solution was 0.072 mM under the sodium formate addition conditions, which was increased by 0.046 mM as compared with the sodium formate non-addition conditions.

### [Example 16]

The TCMT, SahH, FtfL, Fch, MtdA-enhanced strain prepared in Example 12(B) was evaluated for 3-methyltyrosine production as follows.

In this Example, the 3-methyltyrosine production was evaluated in the same manner as in Example 13 except that HMS174(DE3)/pET_P_{T7}-TCMT/pCDF_P_{T7}-sahH-ADO1/pCOLA_P_{T7}-ftfL-fch-mtdA prepared in Example 12(B) was used.

As a result, a 3-methyltyrosine concentration of the reaction solution was 0.078 mM under the sodium formate addition conditions, which was increased by 0.036 mM as compared with the sodium formate non-addition conditions.

### [Comparative Example 9]

The expression of TCMT gene was enhanced in *E*. *coli* HMS174(DE3) strain as follows.

*E. coli* HMS174(DE3) strain (Novagen) was transformed with the plasmid pET_P_{T7}-TCMT constructed in Example 9(A), pCDF Duet-1 (Novagen), and pCOLA Duet-1 (Novagen). The obtained strain was designated as HMS174(DE3)/pET_P_{T7}-TCMT.

### [Comparative Example 10]

The TCMT-enhanced strain prepared in Comparative Example 9 was evaluated for 3-methyltyrosine production as follows.

In this Comparative Example, the 3-methyltyrosine production was evaluated in the same manner as in Example 13 except that HMS174(DE3)/pET_P_{T7}-TCMT prepared in Comparative Example 9 was used.

As a result, a 3-methyltyrosine concentration of the reaction solution was 0.058 mM under the sodium formate addition conditions.

The results of Examples 13 to 16 were summarized in Table 3. In Examples 13, 14, 15, and 16, the concentrations of accumulated 3-methyltyrosine under sodium formate addition conditions were drastically increased to 2.3 times, 4.1 times, 2.8 times, and 1.9 times, respectively, as compared to those under sodium formate non-addition conditions. On the other hand, Comparative Example 10 exhibited the lowest concentration of accumulated 3-methyltyrosine under sodium formate addition conditions.

In Examples 14 and 16, the concentration of accumulated 3-methyltyrosine under sodium formate addition conditions was increased by 15% and 8%, respectively, as compared with the results of Examples 13 and 15, respectively.

These results demonstrated that the enhancement of the expression of Mtn and LuxS as well as the expression of Prs and Apt or the expression of SahH as well as the expression of ADO1 improve SAM regeneration efficiency and can further accelerate the methylation reaction.

Specifically, the method of the present invention was found able to efficiently regenerate SAM, to accelerate methylation reaction, and to increase the amount of an arbitrary methyl compound produced, by using formic acid.

**[Table 3]**

| | Microbial strain | Source of methyl group supply | Concentration of accumulated 3-methyltyrosine [mM] | Amount of increase in concentration of accumulated 3-methyltyrosine as compared with formic acid non-addition conditions [mM] | Relative value of concentration of accumulated 3-methyltyrosine as compared with formic acid non-addition conditions |
|---|---|---|---|---|---|
| Example 13 | HMS174 (DE3)/pET_P_{T7}-TCMT/pCDF_P_{T7}-mtn-1uxS/pCOLA-P_{T7}-ftfL-fch-mtdA | Formic acid | 0.162 | 0.093 | 2.3 |
| Example 14 | HMS174 (DE3)/pET_P_{T7}-TCMT/pCDF_P_{T7}-mtn-1uxS-prs-apt/pCOLA_P_{T7}-ftfL-fch-mtdA | Formic acid | 0.187 | 0.141 | 4.1 |
| Example 15 | HMS174 (DE3)/pET_P_{T7}-TCMT/pCDF_P_{T7}-sahH/pCOLA_P_{T7}-ftfL-fch-mtdA | Formic acid | 0.072 | 0.046 | 2.8 |
| Example 16 | HMS174 (DE3)/pET_P_{T7}-TCMT/pCDF_P_{T7}-sahH-ADO1/pCOLA_P_{T7}-ftfL-fch-mtdA | Formic acid | 0.078 | 0.036 | 1.9 |

### [Example 17]

The expression of EgtB gene, EgtD gene, EgtE gene, mtn gene, luxS gene, ftfL gene, fch gene, and mtdA gene was enhanced in *E. coli* HMS174(DE3) strain as follows.

### (A) Construction of EgtBDE enhancement plasmid

A DNA having a nucleotide sequence (sequence with recognition sequences of restriction enzymes Nco I and EcoR I added to the 5'- and 3'-terminal sides, respectively) comprising *Methylobacterium pseudosasicola-*derived EgtB gene encoding hercynyl cysteine S-oxide synthase and *Mycolicibacterium smegmatis*-derived EgtD gene and EgtE gene encoding L-histidine Nα-methyltransferase and hercynyl cysteine S-oxide lyase, respectively, was synthesized (nucleotide sequence of synthetic DNA: SEQ ID NO: 8). The nucleotide sequences of the EgtB gene, the EgtD gene, and the EgtE gene were each designed as a codon-optimized sequence for efficient expression in *E. coli.* The synthesized DNA was inserted to restriction enzyme sites Nco I and EcoR I of an expression vector pET Duet-1 for *E. coli* (Novagen) and linked downstream of T7 promoter. The constructed plasmid was designated as pET_P_{T17}-EgtBDE.

### (B) Preparation of EgtBDE, Mtn, LuxS, FtfL, Fch, MtdA-enhanced strain

*E. coli* HMS174(DE3) strain (Novagen) was transformed with the plasmid pET_P_{T7}-EgtBDE constructed in Example 17(A), the plasmid pCOLA_P_{T7}-ftfL-fch-mtdA constructed in Example 1(B), and the plasmid pCDF_P_{T7}-mtn-luxS constructed in Example 2(A). The obtained strain was designated as HMS174(DE3)/pET_P_{T7}-EgtBDE/pCDF_P_{T7}-mtn-luxS/pCOLA_P_{T7}-ftfL-fch-mtdA.

### [Example 18]

The expression of EgtB gene, EgtD gene, EgtE gene, mtn gene, luxS gene, Prs gene, Apt gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), the plasmid pCOLA_P_{T7}-ftfL-fch-mtdA constructed in Example 1(B), and the plasmid pCDF_P_{T7}-mtn-luxS-prs-apt constructed in Example 10(A). The obtained strain was designated as HMS174(DE3)/pET_P_{T7}-EgtBDE/pCDF_P_{T7}-mtn-luxS-prs-apt/pCOLA_P_{T7}-ftfL-fch-mtdA.

### [Example 19]

The EgtBDE, Mtn, LuxS, FtfL, Fch, MtdA-enhanced strain prepared in Example 17(B) was evaluated for hercynine and ergothioneine production as follows.

HMS174(DE3)/pET_P_{T7}-EgtBDE/pCDF_P_{T7}-mtn-luxS/pCOLA_P_{T7}-ftfL-fch-mtdA prepared in Example 17(B) was cultured overnight in LB medium (16 g/L Bacto-trypton, 10 g/L Yeast extract, 5 g/L NaCl) comprising 50 µg/mL carbenicillin, 50 µg/mL spectinomycin, and 50 µg/mL kanamycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 50 µg/mL kanamycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM TriS-HCl pH 8.0, 20 mM MgSO₄, 20 mM glucose) comprising 20 mM sodium formate, 100 µg/mL carbenicillin, 100 µg/mL spectinomycin, 100 µg/mL kanamycin, and 1 mM IPTG. 1 mL of the cell suspension was added to a 14 mL tube and mixed with 1 mL of a substrate solution comprising 10 mM L-histidine and 10 mM L-cysteine serving as a substrate to start ergothioneine synthesis reaction comprising methylation. The reaction solution was shaken at 200 rpm at 30°C for 48 hours.

In order to make sure that methylation reaction could be accelerated by efficient regeneration of methyl group carbon of SAM, a test conducted under the same conditions as above except that sodium formate serving as a source of methyl group carbon supply was not added was also carried out, and the obtained results were compared with the results of the test conducted under the sodium formate addition conditions.

The obtained reaction solution was centrifuged at 3000 × g for 5 minutes, and a supernatant was then collected and subjected to LC-MS analysis. The conditions of LC-MS analysis are shown in Table 4.

As a result of measurement, a hercynine concentration and an ergothioneine concentration of the reaction solution were 0.047 mM and 0.078 mM, respectively, under the sodium formate addition conditions, which were increased by 0.033 mM and 0.039 mM, respectively, as compared with the sodium formate non-addition conditions. As a result, the total concentration of accumulated hercynine and accumulated ergothioneine was 0.125 mM under the sodium formate addition conditions, which was increased by 0.073 mM as compared with the sodium formate non-addition conditions.

**[Table 4]**

| | |
|---|---|
| Apparatus | UPLC: Thermo Vanquish UHPLC System Chromeleon 7.2 |
| | MS: Thermo Q Exactive |
| Column | InfinityLab Poroshell 120 HILIC-Z 2.1 mm × 150 mm manufactured by Agilent Technologies, Inc., particle size: 2.7 µm |
| Column temperature | 30°C |
| Mobile phase | (A) 20 mM ammonium formate pH3.0 |
| | (B) Acetonitrile/20 mM ammonium formate pH3.0 |
| Detection | PDA (220-700 nm), MS (ESI, Positive) |
| Flow rate | 0.8 mL/min |
| Elution | 0 min 100% B→8 min 76% B→8.5 min 70% B→8.5 min 100% B→14.75 min 100% B |

### [Example 20]

The EgtBDE, Mtn, LuxS, Prs, Apt, FtfL, Fch, MtdA-enhanced strain prepared in Example 18 was evaluated for hercynine and ergothioneine production as follows.

In this Example, the hercynine and ergothioneine production was evaluated in the same manner as in Example 19 except that HMS174(DE3)/pET_P_{T7}-EgtBDE/pCDF_P_{T7}-mtn-luxS-prs-apt/pCOLA_P_{T7}-ftfL-fch-mtdA prepared in Example 18 was used.

As a result, the total concentration of accumulated hercynine and accumulated ergothioneine in the reaction solution was 0.228 mM under the sodium formate addition conditions, which was increased by 0.014 mM as compared with the sodium formate non-addition conditions.

### [Comparative Example 11]

The expression of EgtB gene, EgtD gene, and EgtE gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), pCDF Duet-1 (Novagen), and pCOLA Duet-1 (Novagen). The obtained strain was designated as HMS174(DE3)/pET_P_{T7}-EgtBDE.

### [Comparative Example 12]

The expression of EgtB gene, EgtD gene, EgtE gene, mtn gene, and luxS gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), the plasmid pCDF_P_{T7}-mtn-luxS constructed in Example 2(A), and pCOLA Duet-1 (Novagen). The obtained strain was designated as HMS174(DE3)/pET_P_{T7}-EgtBDE/pCDF_P_{T7}-mtn-luxS.

### [Comparative Example 13]

The expression of EgtB gene, EgtD gene, EgtE gene, mtn gene, luxS gene, Prs gene, and Apt gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), the plasmid pCDF_P_{T7}-mtn-luxS-prs-apt constructed in Example 10(A), and pCOLA Duet-1 (Novagen). The obtained strain was designated as HMS174(DE3)/pET_P_{T7}-EgtBDE/pCDF_P_{T7}-mtn-luxS-prs-apt.

### [Comparative Example 14]

The EgtBDE-enhanced strain prepared in Comparative Example 11 was evaluated for hercynine and ergothioneine production as follows.

In this Comparative Example, the hercynine and ergothioneine production was evaluated in the same manner as in Example 19 except that HMS174(DE3)/pET_P_{T7}-EgtBDE prepared in Comparative Example 11 was used.

As a result, neither a hercynine concentration nor an ergothioneine concentration of the reaction solution was increased as compared with the sodium formate non-addition conditions, and the total concentration of accumulated hercynine and accumulated ergothioneine was not increased either.

### [Comparative Example 15]

The EgtBDE, Mtn, LuxS-enhanced strain prepared in Comparative Example 12 was evaluated for hercynine and ergothioneine production as follows.

In this Comparative Example, the hercynine and ergothioneine production was evaluated in the same manner as in Example 19 except that HMS174(DE3)/pET_P_{T7}-EgtBDE/pCDF_P_{T7}-mtn-luxS prepared in Comparative Example 12 was used.

As a result, a hercynine concentration and an ergothioneine concentration of the reaction solution were equivalent or not increased as compared with the sodium formate non-addition conditions, and the total concentration of accumulated hercynine and accumulated ergothioneine was not increased either.

### [Comparative Example 16]

The EgtBDE, Mtn, LuxS, Prs, Apt -enhanced strain prepared in Comparative Example 13 was evaluated for hercynine and ergothioneine production as follows.

In this Comparative Example, the hercynine and ergothioneine production was evaluated in the same manner as in Example 19 except that HMS174(DE3)/pET_P_{T7}-EgtBDE/pCDF_P_{T7}-mtn-luxS-prs-apt prepared in Comparative Example 13 was used.

As a result, the total concentration of accumulated hercynine and accumulated ergothioneine in the reaction solution was 0.123 mM under the sodium formate addition conditions.

The results of Examples 19 and 20 are summarized in Table 5. In order to evaluate methylation reaction efficiency, the total concentration of accumulated hercynine and ergothioneine was calculated and compared. As a result, in Example 19, the total concentration of accumulated compounds under sodium formate addition conditions was drastically increased to 2.4 times as compared to that under sodium formate non-addition conditions. On the other hand, both Comparative Examples 14 and 15 exhibited no increase in total concentration of accumulated compounds under sodium formate addition conditions.

In Example 20, the total concentration of accumulated compounds under sodium formate addition conditions was increased to 1.1 times as compared to that under sodium formate non-addition conditions and was 1.9 times that under sodium formate addition conditions in Comparative Example 16.

These results demonstrated that the use of formic acid under conditions with enhanced expression of FtfL, Fch, and MtdA allows more efficient regeneration of SAM and can accelerate the methylation reaction and increase the amount of an arbitrary methyl compound produced, as compared with the case of using no formic acid or the case of using formic acid under conditions without enhanced expression of the enzymes.

In Example 20, the total concentration of accumulated compounds under sodium formate addition conditions was 1.8 times that of the results of Example 19.

These results demonstrated that the enhancement of the expression of Mtn, LuxS, Prs, and Apt improves SAM regeneration efficiency and can further accelerate the methylation reaction.

Specifically, the method of the present invention was found able to efficiently regenerate SAM, to accelerate methylation reaction, and to increase the amount of an arbitrary methyl compound produced, by using formic acid.

**[Table 5]**

| | Microbial strain | Source of methyl group supply | Total concentration of accumulated hercynine (Her) and ergothioneine (Egt) [mM] | Amount of increase in total concentration of accumulated Her and Egt as compared with formic acid non-addition conditions [mM] | Relative value of total concentration of accumulated Her and Egt as compared with formic acid non-addition conditions |
|---|---|---|---|---|---|
| Example 19 | HMS174 (DE3)/pET_P_{T7}-EgtBDE/pCDF_P_{T7}-mtn-1uxS/pCOLA_P_{T7}-ftfL-fch-mtdA | Formic acid | 0.125 | 0.072 | 2.4 |
| Example 20 | HMS174 (DE3)/pET_P_{T7}-EgtBDE/pCDF_P_{T7}-mtn-luxS-prs-apt/pCOLA_P_{T7}-ftfL-fch-mtdA | Formic acid | 0.228 | 0.014 | 1.1 |

### [Example 21]

The expression of ASMT gene, mtn gene, luxS gene, ftfL gene, fch gene, and mtdA gene was enhanced in *E. coli* HMS174(DE3) strain as follows.

### (A) Construction of ASMT enhancement plasmid

A DNA having a nucleotide sequence (sequence with recognition sequences of restriction enzymes Nco I and Bgl II added to the 5'- and 3'-terminal sides, respectively) comprising *Homo sapiens*-derived ASMT gene encoding acetylserotonin O-methyltransferase was synthesized (nucleotide sequence of synthetic DNA: SEQ ID NO: 9). The nucleotide sequence of the ASMT gene was designed as a codon-optimized sequence for efficient expression in *E. coli* with alanine at position 258 mutated to glutamic acid. The synthesized DNA was inserted to restriction enzyme sites Nco I and Bgl II of an expression vector pET Duet-1 for *E. coli* (Novagen) and linked downstream of T7 promoter. The constructed plasmid was designated as pET_P_{T7}-ASMT.

### (B) Preparation of ASMT, Mtn, LuxS, FtfL, Fch, MtdA-enhanced strain

*E. coli* HMS174(DE3) strain (Novagen) was transformed with the plasmid pET_P_{T7}-ASMT constructed in Example 21(A), the plasmid pCOLA_P_{T7}-ftfL-fch-mtdA constructed in Example 1(B), and the plasmid pCDF_P_{T7}-mtn-luxS constructed in Example 2(A). The obtained strain was designated as HMS174(DE3)/pET_P₁₇-ASMT/pCDF_P₁₇-mtn-luxS/pCOLA_P_{T7}-ftfL-fch-mtdA.

### [Example 22]

The expression of ASMT gene, mtn gene, luxS gene, Prs gene, Apt gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-ASMT constructed in Example 21(A), the plasmid pCOLA_P_{T7}-ftfL-fch-mtdA constructed in Example 1(B), and the plasmid pCDF_P_{T7}-mtn-luxS-prs-apt constructed in Example 10(A). The obtained strain was designated as HMS174(DE3)/pET_P_{T7}-ASMT/pCDF_P_{T7}-mtn-luxS-prs-apt/pCOLA_P_{T7}-ftfL-fch-mtdA.

### [Example 23]

The ASMT, Mtn, LuxS, FtfL, Fch, MtdA-enhanced strain prepared in Example 21(B) was evaluated for melatonin production as follows.

HMS174(DE3)/pET_P_{T7}-ASMT/pCDF_P_{T7}-mtn-luxS/pCOLA_P_{T7}-ftfL-fch-mtdA prepared in Example 21(B) was cultured overnight in LB medium (16 g/L Bacto-trypton, 10 g/L Yeast extract, 5 g/L NaCl) comprising 50 µg/mL carbenicillin, 50 µg/mL spectinomycin, and 50 µg/mL kanamycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 50 µg/mL kanamycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 20 mM sodium formate, 100 µg/mL carbenicillin, 100 µg/mL spectinomycin, 100 µg/mL kanamycin, and 1 mM IPTG. 1 mL of the cell suspension was added to a 14 mL tube and mixed with 1 mL of a substrate solution comprising 50 mM N-acetylserotonin serving as a substrate to start melatonin synthesis reaction comprising methylation. The reaction solution was shaken at 200 rpm at 30°C for 48 hours.

In order to make sure that methylation reaction could be accelerated by efficient regeneration of methyl group carbon of SAM, a test conducted under the same conditions as above except that sodium formate serving as a source of methyl group carbon supply was not added was also carried out, and the obtained results were compared with the results of the test conducted under the sodium formate addition conditions.

100 µL of the obtained reaction solution was mixed with 100 µL of methanol, and a supernatant was collected after centrifugation at 3000 × g for 5 minutes and subjected to LC-MS analysis. The conditions of LC-MS analysis are shown in Table 6.

As a result of measurement, a melatonin concentration of the reaction solution was 0.144 mM under the sodium formate addition conditions, which was increased by 0.036 mM as compared with the sodium formate non-addition conditions.

**[Table 6]**

| | |
|---|---|
| Apparatus | UPLC: Thermo Vanquish UHPLC System Chromeleon 7.2 |
| | MS: Thermo Q Exactive |
| Column | ACQUITY UPLC BEH C18 2.1 mm × 50 mm manufactured by Waters Corp., particle size: 1.7 µm |
| Column temperature | 42°C |
| Mobile phase | (A) 0.1% formic acid |
| | (B) Methanol |
| Detection | PDA (220-700 nm), MS (ESI, Positive) |
| Flow rate | 0-8 min: 0.2 ml/min→8-9.5 min: 0.5 ml/min→9.5-10 min: 0.2 ml/min |
| Elution | 0 min 5% β→5 min 40% B→7 min 100% B→8 min 100% B→8 min 5% B→9.5 min 5% B→10 min 5% B |

### [Example 24]

The ASMT, Mtn, LuxS, Prs, Apt, FtfL, Fch, MtdA-enhanced strain prepared in Example 22 was evaluated for melatonin production as follows.

In this Example, the melatonin production was evaluated in the same manner as in Example 23 except that HMS174(DE3)/pET_P_{T7}-ASMT/pCDF_P_{T7}-mtn-luxS-prs-apt/pCOLA_P_{T7}-ftfL-fch-mtdA prepared in Example 22 was used.

As a result, a melatonin concentration of the reaction solution was 0.153 mM under the sodium formate addition conditions, which was increased by 0.031 mM as compared with the sodium formate non-addition conditions.

### [Comparative Example 17]

The expression of ASMT gene was enhanced in *E*. *coli* HMS174(DE3) strain as follows.

*E. coli* HMS174(DE3) strain (Novagen) was transformed with the plasmid pET_P_{T7}-ASMT constructed in Example 21(A), pCDF Duet-1 (Novagen), and pCOLA Duet-1 (Novagen). The obtained strain was designated as HMS174(DE3)/pET_P_{T7}-ASMT.

### [Comparative Example 18]

The ASMT-enhanced strain prepared in Comparative Example 17 was evaluated for melatonin production as follows.

In this Comparative Example, the melatonin production was evaluated in the same manner as in Example 23 except that HMS174(DE3)/pET_P_{T7}-ASMT prepared in Comparative Example 17 was used.

As a result, a melatonin concentration of the reaction solution was 0.075 mM under the sodium formate addition conditions, which was not increased as compared with the sodium formate non-addition conditions.

The results of Examples 23 and 24 were summarized in Table 7. In Examples 23 and 24, the concentration of accumulated melatonin under sodium formate addition conditions was increased by 33% and 25%, respectively, as compared with sodium formate non-addition conditions. On the other hand, Comparative Example 18 exhibited no increase in melatonin accumulation under sodium formate addition conditions.

These results demonstrated that the use of formic acid under conditions with enhanced expression of FtfL, Fch, and MtdA allows more efficient regeneration of SAM and can accelerate the methylation reaction and increase the amount of an arbitrary methyl compound produced, as compared with the case of using no formic acid or the case of using formic acid under conditions without enhanced expression of the enzymes.

In Examples 23 and 24, the concentrations of accumulated melatonin under sodium formate addition conditions were increased to 1.9 times and 2.0 times, respectively, as compared to the results of Comparative Example 18.

These results demonstrated that the enhancement of the expression of Mtn, LuxS, Prs, and Apt improves SAM regeneration efficiency and can further accelerate the methylation reaction.

Specifically, the method of the present invention was found able to efficiently regenerate SAM, to accelerate methylation reaction, and to increase the amount of an arbitrary methyl compound produced, by using formic acid.

**[Table 7]**

| | Microbial strain | Source of methyl group supply | Concentration of accumulated melatonin [mM] | Amount of increase in concentration of accumulated melatonin as compared with formic acid non-addition conditions [mM] | Rate of increase in concentration of accumulated melatonin as compared with formic acid non-addition conditions (%) |
|---|---|---|---|---|---|
| Example 23 | HMS174 (DE3)/pET_P_{T7}-ASMT/pCDF_P_{T7}-mtn-luxS/pCOLA_P_{T7}-ftfL-fch-mtdA | Formic acid | 0.144 | 0.036 | 33 |
| Example 24 | HMS174 (DE3)/pET_P_{T7}-ASMT/pCDF_P_{T7}-mtn-1uxS-prs-apt/pCOLA_P_{T7}-ftfL-fch-mtdA | Formic acid | 0.153 | 0.031 | 25 |

### [Example 25]

The expression of sfmM2 gene, mdh2 gene, ftfL gene, fch gene, and mtdA gene was enhanced in *E. coli* HMS174(DE3) strain as follows.

### (A) Construction of Mdh2 enhancement plasmid

A DNA having a nucleotide sequence (sequence with recognition sequences of restriction enzymes Nde I and Aat II added to the 5'- and 3'-terminal sides, respectively) comprising *Cupriavidus necator*-derived mdh2 gene was synthesized (nucleotide sequence of synthetic DNA: SEQ ID NO: 10). The nucleotide sequence comprising the mdh2 gene was designed as a codon-optimized sequence for efficient expression in *E. coli* with alanine at position 26 mutated to valine, alanine at position 31 mutated to valine, and alanine 169 mutated to valine. The synthesized DNA was inserted to restriction enzyme sites Nde I and Aat II of an expression vector pACYC Duet-1 for *E. coli* (Novagen) and linked downstream of T7 promoter. The constructed plasmid was designated as pACYC_pT7-mdh2.

### (B) Preparation of SfmM2, Mdh2, FtfL, Fch, MtdA-enhanced strain

*E. coli* HMS174(DE3) strain (Novagen) was transformed with the plasmid pET_P_{T7}-sfmM2 constructed in Example 1(A), the plasmid pACYC_P_{T7}-mdh2 constructed in Example 25(A), the plasmid pCOLA_P_{T7}-ftfL-fch-mtdA constructed in Example 1(B), and pCDF Duet-1(Novagen). The obtained strain was designated as HMS174(DE3)/pET_P_{T7}-sfmM2/pACYC_P_{T7}-mdh2/pCOLA_P_{T7}-ftfL-fch-mtdA.

### [Example 26]

The expression of sfmM2 gene, mdh2 gene, luxS gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pACYC_P_{T7}-mdh2 constructed in Example 25(A), the plasmid pCOLA_P_{T7}-ftfL-fch-mtdA constructed in Example 1(B), and the plasmid pCDF_P_{T7}-luxS constructed in Example 2(B). The obtained strain was designated as HMS174(DE3)/pET_P_{T7}-sfmM2/pACYC_P_{T7}-mdh2/pCDF_P_{T7}-luxS/pCOLA_P_{T7}-ftfL-fch-mtdA.

### [Example 27]

The expression of sfmM2 gene, mdh2 gene, mtn gene, luxS gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pACYC_P_{T7}-mdh2 constructed in Example 25(A), the plasmid pCOLA_P_{T7}-ftfL-fch-mtdA constructed in Example 1(B), and the plasmid pCDF_P_{T7}-mtn-luxS constructed in Example 2(A). The obtained strain was designated as HMS174(DE3)/pET_P_{T7}-sfmM2/pACYC_P_{T7}-mdh2/pCDF_P_{T7}-mtn-luxS/pCOLA_P_{T7}-ftfL-fch-mtdA.

### [Example 28]

The SfmM2, Mdh2, FtfL, Fch, MtdA-enhanced strain prepared in Example 25(B) was evaluated for 3-methyltyrosine production as follows.

HMS174(DE3)/pET_P_{T7}-sfmM2/pACYC_P_{T17}-mdh2/pCOLA_P_{T7}-ftfL-fch-mtdA prepared in Example 25(B) was cultured overnight in LB medium (16 g/L Bacto-trypton, 10 g/L Yeast extract, 5 g/L NaCl) comprising 50 µg/mL carbenicillin, 34 µg/mL chloramphenicol, 50 µg/mL spectinomycin, and 50 µg/mL kanamycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 34 µg/mL chloramphenicol, 50 µg/mL spectinomycin, and 50 µg/mL kanamycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 10 in a reaction buffer (100 mM Tris-HCl pH 8.0, 20 mM MgSO₄, 20 mM glucose) comprising 100 mM methanol, 100 µg/mL carbenicillin, 68 µg/mL chloramphenicol, 100 µg/mL spectinomycin, 100 µg/mL kanamycin, and 1 mM IPTG. 1 mL of the cell suspension was added to a 14 mL tube and mixed with 1 mL of a 10 mM L-tyrosine solution (dissolved in 0.1 N hydrochloric acid) serving as a substrate to start methylation reaction. The reaction solution was shaken at 200 rpm at 30°C for 24 hours. In order to make sure that methylation reaction could be accelerated by efficient regeneration of methyl group carbon of SAM, a test conducted under the same conditions as above except that methanol serving as a source of methyl group carbon supply was not added was also carried out, and the obtained results were compared with the results of the test conducted under the methanol addition conditions.

The obtained reaction solution was subjected to LC-MS analysis in the same manner as in Example 5. As a result of measurement, a 3-methyltyrosine concentration of the reaction solution was 0.042 mM under the methanol addition conditions, which was increased by 0.010 mM as compared with the methanol non-addition conditions.

### [Example 29]

The SfmM2, Mdh2, LuxS, FtfL, Fch, MtdA-enhanced strain prepared in Example 26 was evaluated for 3-methyltyrosine production as follows.

In this Example, the 3-methyltyrosine production was evaluated in the same manner as in Example 28 except that HMS174(DE3)/pET_P_{T7}-sfmM2/pACYC_P_{T7}-mdh2/pCDF_P_{T7}-luxS/pCOLA_P_{T7}-ftfL-fch-mtdA prepared in Example 26 was used.

As a result, a 3-methyltyrosine concentration of the reaction solution was 0.080 mM under the methanol addition conditions, which was increased by 0.014 mM as compared with the methanol non-addition conditions.

### [Example 30]

The SfmM2, Mdh2, Mtn, LuxS, FtfL, Fch, MtdA-enhanced strain prepared in Example 27 was evaluated for 3-methyltyrosine production as follows.

In this Example, the 3-methyltyrosine production was evaluated in the same manner as in Example 28 except that HMS174(DE3)/pET_P_{T7}-sfmM2/pACYC_P_{T7}-mdh2/pCDF_P_{T7}-mtn-luxS/pCOLA_P_{T7}-ftfL-fch-mtdA prepared in Example 27 was used.

As a result, a 3-methyltyrosine concentration of the reaction solution was 0.158 mM under the methanol addition conditions, which was increased by 0.059 mM as compared with the methanol non-addition conditions.

### [Comparative Example 19]

The expression of sfmM2 gene was enhanced in *E*. *coli* HMS174(DE3) strain as follows.

*E. coli* HMS174(DE3) strain (Novagen) was transformed with the plasmid pET_P_{T7}-sfmM2 constructed in Example 1(A), pACYC Duet-1(Novagen), pCDF Duet-1(Novagen), and pCOLA Duet-1 (Novagen). The obtained strain was designated as HMS174(DE3)/pET_P_{T7}-sfmM2.

### [Comparative Example 20]

The expression of sfmM2 gene and mdh2 gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pACYC_P_{T7}-mdh2 constructed in Example 25(A), pCDF Duet-1(Novagen), and pCOLA Duet-1(Novagen). The obtained strain was designated as HMS174(DE3)/pET _P_{T7}-sfmM2/pACYC_P_{T7}-mdh2.

### [Comparative Example 21]

The expression of sfmM2 gene, mtn gene, and luxS gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-mtn-luxS constructed in Example 2(A), pACYC Duet-1(Novagen), and pCOLA Duet-1(Novagen). The obtained strain was designated as HMS174(DE3)/pET_P_{T7}-sfmM2/pCDF_P_{T7}-mtn-luxS.

### [Comparative Example 22]

The expression of sfmM2 gene, mdh2 gene, mtn gene, and luxS gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pACYC_P_{T7}-mdh2 constructed in Example 25(A), the plasmid pCDF_P_{T7}-mtn-luxS constructed in Example 2(A), and pCOLA Duet-1(Novagen). The obtained strain was designated as HMS174(DE3)/pET_P_{T7}-sfmM2/pACYC_P_{T7}-mdh2/pCDF_P_{T7}-mtn-luxS.

### [Comparative Example 23]

The SfmM2-enhanced strain prepared in Comparative Example 19 was evaluated for 3-methyltyrosine production as follows.

In this Comparative Example, the 3-methyltyrosine production was evaluated in the same manner as in Example 28, except that HMS174(DE3)/pET_P_{T7}-sfmM2 prepared in Comparative Example 19 was used.

As a result, a 3-methyltyrosine concentration of the reaction solution was not increased as compared with the methanol non-addition conditions.

### [Comparative Example 24]

The SfmM2, Mdh2-enhanced strain prepared in Comparative Example 20 was evaluated for 3-methyltyrosine production as follows.

In this Comparative Example, the 3-methyltyrosine production was evaluated in the same manner as in Example 28 except that HMS174(DE3)/pET_P_{T7}-sfmM2/pACYC_P_{T7}-mdh2 prepared in Comparative Example 20 was used.

As a result, a 3-methyltyrosine concentration of the reaction solution was not increased as compared with the methanol non-addition conditions.

### [Comparative Example 25]

The SfmM2, Mtn, LuxS-enhanced strain prepared in Comparative Example 21 was evaluated for 3-methyltyrosine production as follows.

In this Comparative Example, the 3-methyltyrosine production was evaluated in the same manner as in Example 28 except that HMS174(DE3)/pET_P_{T7}-sfmM2/pCDF_P_{T7}-mtn-luxS prepared in Comparative Example 21 was used.

As a result, a 3-methyltyrosine concentration of the reaction solution was not increased as compared with the methanol non-addition conditions.

### [Comparative Example 26]

The SfmM2, Mdh2, Mtn, LuxS-enhanced strain prepared in Comparative Example 22 was evaluated for 3-methyltyrosine production as follows.

In this Comparative Example, the 3-methyltyrosine production was evaluated in the same manner as in Example 28 except that HMS174(DE3)/pET_P_{T7}-stmM2/pACYC_P_{T7}-mdh2/pCDF_P_{T7}-mtn-luxS prepared in Comparative Example 22 was used.

As a result, a 3-methyltyrosine concentration of the reaction solution was not increased as compared with the methanol non-addition conditions.

The results of Examples 28 to 30 were summarized in Table 8. In Examples 28, 29, and 30, the concentration of accumulated 3-methyltyrosine under methanol addition conditions was increased by 31%, 21%, and 60%, respectively, as compared with methanol non-addition conditions. On the other hand, Comparative Examples 23, 24, 25, and 26 exhibited no increase in 3-methyltyrosine accumulation under methanol addition conditions.

These results demonstrated that the use of methanol under conditions with enhanced expression of Mdh2, FtfL, Fch, and MtdA allows more efficient regeneration of SAM and can accelerate the methylation reaction and increase the amount of an arbitrary methyl compound produced, as compared with the case of using no methanol or the case of using methanol under conditions without enhanced expression of the enzymes.

In Examples 29 and 30, the concentrations of accumulated 3-methyltyrosine under methanol addition conditions were 1.9 times and 3.8 times, respectively, those in Example 28.

These results demonstrated that the enhancement of the expression of LuxS or the expression of Mtn and LuxS improves SAM regeneration efficiency and can further accelerate the methylation reaction and increase the amount of an arbitrary methyl compound produced.

Specifically, the method of the present invention was found to efficiently regenerate SAM, to accelerate methylation reaction, and to increase the amount of an arbitrary methyl compound produced, by using methanol.

**[Table 8]**

| | Microbial strain | Source of methyl group supply | Concentration of accumulated 3-methyltyrosine [mM] | Amount of increase in concentration of accumulated 3-methyltyrosine as compared with formic acid non-addition conditions [mM] | Rate of increase in concentration of accumulated 3-methyltyrosine as compared with formic acid non-addition conditions (%) |
|---|---|---|---|---|---|
| Example 28 | HMS174 (DE3)/pET_P_{T7}-sfmM2/pACYC_P_{T7}-mdh2/pCOLA_P_{F7}-ftfL-fch-mtdA | Methanol | 0.042 | 0.010 | 31 |
| Example 29 | HMS174 (DE3)/pET_P_{T7}-sfmM2/pACYC_P_{T7}-mdh2/pCDF_P_{T7}-luxS/pCOLA_P_{T7}-ftfL-fch-mtdA | Methanol | 0.080 | 0.014 | 21 |
| Example 30 | HMS174 (DE3) /pET_P_{T7}-sfmM2/pACYC_P_{T7}-mdh2/pCDF_P_{T7}-mtn-1uxS/pCOLA_P_{T7}-ftfL-fch-mtdA | Methanol | 0.158 | 0.128 | 60 |

### [Example 31]

The expression of EgtB gene, EgtD gene, EgtE gene, mdh2 gene, mtn gene, luxS gene, ftfL gene, fch gene, and mtdA gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), the plasmid pACYC_P_{T7}-mdh2 constructed in Example 25(A), the plasmid pCDF_P_{T7}-mtn-luxS constructed in Example 2(A), and the plasmid pCOLA_P_{T7}-ftfL-fch-mtdA constructed in Example 1(B). The obtained strain was designated as HMS174(DE3)/pET_P_{T7}-EgtBDE/pACYC_P_{T7}-mdh2/pCDF_P_{T7}-mtn-luxS/pCOLA_P_{T7}-ftfL-fch-mtdA.

### [Example 32]

The EgtBDE, Mdh2, Mtn, LuxS, FtfL, Fch, MtdA-enhanced strain prepared in Example 31 was evaluated for hercynine and ergothioneine production as follows.

HMS174(DE3)/pET_P_{T7}-EgtBDE/pACYC_P_{T7}-mdh2/pCDF_P_{T7}-mtn-luxS/pCOLA_P_{T7}-ftfL-fch-mtdA prepared in Example 31 was cultured overnight in LB medium (16 g/L Bacto-trypton, 10 g/L Yeast extract, 5 g/L NaCl) comprising 50 µg/mL carbenicillin, 34 µg/mL chloramphenicol, 50 µg/mL spectinomycin, and 50 µg/mL kanamycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 34 µg/mL chloramphenicol, 50 µg/mL spectinomycin, and 50 µg/mL kanamycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 100 mM methanol, 100 µg/mL carbenicillin, 68 µg/mL chloramphenicol, 100 µg/mL spectinomycin, 100 µg/mL kanamycin, and 1 mM IPTG. 1 mL of the cell suspension was added to a 14 mL tube and mixed with 1 mL of a substrate solution comprising 10 mM L-histidine and 10 mM L-cysteine serving as a substrate to start ergothioneine synthesis reaction comprising methylation. The reaction solution was shaken at 200 rpm at 30°C for 48 hours.

In order to make sure that methylation reaction could be accelerated by efficient regeneration of methyl group carbon of SAM, a test conducted under the same conditions as above except that methanol serving as a source of methyl group carbon supply was not added was also carried out, and the obtained results were compared with the results of the test conducted under the methanol addition conditions.

The obtained reaction solution was subjected to LC-MS analysis in the same manner as in Example 19.

As a result of measurement, a hercynine concentration and an ergothioneine concentration of the reaction solution were 0.011 mM and 0.023 mM, respectively, under the methanol non-addition conditions, whereas these concentrations were 0.011 mM and 0.027 mM, respectively, under the methanol addition conditions. The total concentration of accumulated hercynine and accumulated ergothioneine was 0.039 mM under the methanol addition conditions, which was increased by 0.005 mM as compared with the methanol non-addition conditions.

### [Comparative Example 27]

The expression of EgtB gene, EgtD gene, and EgtE gene was enhanced in *E. coli* HMS174(DE3) strain as follows.

*E. coli* HMS174(DE3) strain (Novagen) was transformed with the plasmid pET_P_{T7}-EgtBDE constructed in Example 17(A), pACYC Duet-1 (Novagen), pCDF Duet-1 (Novagen), and pCOLA Duet-1 (Novagen). The obtained strain was designated as HMS 174(DE3)/pET_P_{T7}-EgtBDE.

### [Comparative Example 28]

The EgtBDE-enhanced strain prepared in Comparative Example 27 was evaluated for hercynine and ergothioneine production as follows.

In this Comparative Example, the hercynine and ergothioneine production was evaluated in the same manner as in Example 32 except that HMS 174(DE3)/pET_P_{T7}-EgtBDE prepared in Comparative Example 27 was used.

As a result, neither a hercynine concentration nor an ergothioneine concentration of the reaction solution was increased as compared with the methanol non-addition conditions, and the total concentration of accumulated hercynine and accumulated ergothioneine was not increased either. The total concentration of accumulated hercynine and accumulated ergothioneine was 0.016 mM under the methanol addition conditions.

The results of Example 32 are summarized in Table 9. In order to evaluate methylation reaction efficiency, the total concentration of accumulated hercynine and ergothioneine was calculated and compared. As a result, in Example 32, the total concentration of accumulated compounds under methanol addition conditions was increased by 15% as compared with methanol non-addition conditions. On the other hand, Comparative Example 28 exhibited no increase in total concentration of accumulated compounds under methanol addition conditions.

These results demonstrated that the use of methanol under conditions with enhanced expression of Mdh2, FtfL, Fch and MtdA allows more efficient regeneration of SAM and can accelerate the methylation reaction and increase the amount of an arbitrary methyl compound produced, as compared with the case of using no methanol or the case of using methanol under conditions without enhanced expression of the enzymes.

In Example 32, the total concentration of accumulated compounds under methanol addition conditions was 2.4 times that of the results of Comparative Example 28.

These results demonstrated that the enhancement of the expression of Mtn and LuxS improves SAM regeneration efficiency and can further accelerate the methylation reaction.

Specifically, the method of the present invention was found to efficiently regenerate SAM, to accelerate methylation reaction, and to increase the amount of an arbitrary methyl compound produced, by using methanol.

**[Table 9]**

| | Microbial strain | Source of methyl group supply | Total concentration of accumulated hercynine (Her) and ergothioneine (Egt) [mM] | Amount of increase in total concentration of accumulated Her and Egt as compared with methanol non-addition conditions [mM] | Rate of increase in total concentration of accumulated Her and Egt as compared with methanol non-addition conditions (%) |
|---|---|---|---|---|---|
| Example 32 | HMS174 (DE3)/pET_P_{T7}-EgtBDE/pACYC _PT7-mdh2/pCDF_P_{T7}-mtn-1uxS/pCOLA_P_{T7}-ftfL-fch-mtdA | Methanol | 0.039 | 0.005 | 15 |

### [Industrial Applicability]

The present invention can provide an industrially useful method for regenerating SAM and method for producing a methyl compound.

### [Free Text of Sequence Listing]

SEQ ID NOs: 1 to 10: synthetic DNA

## Claims

1. A method for producing a methyl compound, comprising the steps of:
in the presence of formic acid or a salt thereof,
(a) producing 5-methyltetrahydrofolate from formic acid or a salt thereof and tetrahydrofolate;
(b) converting S-adenosylhomocysteine, which is produced by a demethylation of S-adenosylmethionine, to homocysteine;
(c) producing methionine by transferring a methyl group of the 5-methyltetrahydrofolate to the homocysteine, and converting the methionine to S-adenosylmethionine; and
(d) performing methylation reaction with S-adenosylmethionine as a methyl group donor to produce a methyl compound.

2. The method according to claim 1, wherein in the step (a), the production of 5-methyltetrahydrofolate from formic acid and tetrahydrofolate is performed in the presence of formate-tetrahydrofolate ligase (FtfL), methenyl-tetrahydrofolate cyclohydrolase (Fch), methylene-tetrahydrofolate dehydrogenase (MtdA), and 5,10-methylene-tetrahydrofolate reductase (MetF).

3. The method according to claim 1, wherein in the step (b), the conversion of S-adenosylhomocysteine to homocysteine is performed in the presence of S-adenosylhomocysteine nucleosidase (Mtn) and S-ribosylhomocysteine lyase (LuxS).

4. The method according to claim 1, wherein in the step (b), the conversion of S-adenosylhomocysteine to homocysteine is performed in the presence of S-adenosylhomocysteine hydrolase (SahH).

5. The method according to claim 1, wherein in the step (b), the demethylation of S-adenosylhomocysteine is performed in the presence of S-adenosylmethionine-dependent methyltransferase.

6. The method according to claim 1, wherein in the step (c), the production of methionine from the homocysteine is performed in the presence of methionine synthase (MetH or MetE), and the conversion of the methionine to S-adenosylmethionine is performed in the presence of methionine adenosyltransferase (MetK).

7. The method according to claim 1, further comprising the step of:
(e) converting methanol to formaldehyde, and converting the formaldehyde to formic acid.

8. The method according to claim 7, wherein in the step (e), the conversion of methanol to formaldehyde is performed in the presence of methanol dehydrogenase (Medh) and/or methanol oxidase (Mox).

9. The method according to claim 7, wherein in the step (e), the conversion of formaldehyde to formic acid is performed in the presence of formaldehyde dehydrogenase (FdhA).

10. The method according to claim 7, wherein in the step (e), the conversion of formaldehyde to formic acid is performed in the presence of spontaneous reaction of formaldehyde with glutathione, and in the presence of S-hydroxymethyl glutathione dehydrogenase (FrmA) and S-formylglutathione hydrolase (FrmB).

11. The method according to claim 1, wherein a cell engineered so as to enhance the activity or expression of S-adenosylmethionine-dependent methyltransferase is used.

12. The method according to claim 11, wherein the cell is at least one selected from the group consisting of an animal cell, an insect cell, a plant cell, and a microorganism.

13. The method according to claim 1, wherein the methyl compound is a derivative of an aromatic amino acid.

14. A method for producing an organic compound, comprising the step of producing the organic compound via a methyl compound produced by a method according to claim 1 as an intermediate.

15. A cell engineered so as to enhance the activity or expression of formate-tetrahydrofolate ligase (FtfL) and S-adenosylmethionine-dependent methyltransferase.

16. The cell according to claim 15, further engineered so as to enhance the activity or expression of methenyl-tetrahydrofolate cyclohydrolase (Fch) and/or methylene-tetrahydrofolate dehydrogenase (MtdA).

17. The cell according to claim 15, further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).

18. The cell according to claim 15, further engineered so as to enhance the activity or expression of at least one or more enzymes selected from the group consisting of ribose-phosphate diphosphokinase (Prs), adenine phosphoribosyltransferase (Apt), and adenosine kinase (ADK).

19. The cell according to claim 15, further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).

20. The cell according to claim 15, further engineered so as to enhance the activity or expression of methanol dehydrogenase (Medh) and/or methanol oxidase (Mox).

21. The cell according to claim 20, further engineered so as to enhance the activity or expression of at least one or more enzymes selected from the group consisting of formaldehyde dehydrogenase (FdhA), S-hydroxymethyl glutathione dehydrogenase (FrmA), and S-formylglutathione hydrolase (FrmB).

22. The cell according to claim 15, wherein the cell is at least one selected from the group consisting of an animal cell, an insect cell, a plant cell, and a microorganism.

23. A method for regenerating S-adenosylmethionine, comprising the steps of:
(a) producing 5-methyltetrahydrofolate from formic acid or a salt thereof and tetrahydrofolate;
(b) converting S-adenosylhomocysteine, which is produced by the demethylation of S-adenosylmethionine, to homocysteine; and
(c) producing methionine by transferring a methyl group of the 5-methyltetrahydrofolate to the homocysteine, and converting the methionine to S-adenosylmethionine.
